# EUROPEAN PATENT SPECIFICATION

(11) **EP 2 872 892 B1**
(45) Date of publication and mention of the grant of the patent: **20.12.2017**
(21) Application number: 12742775.5
(22) Date of filing: 10.07.2012
(51) Int. Cl.: G01N 33/543, B01L 3/00

(54) **FLEXIBLE DNA SENSOR CARRIER AND METHOD**
FLEXIBLE DNS SENSOR TRAGERFORM UND VERFAHREN ZU DESSEN VERWENDUNG
SENSOR ADN SUPPORT FLEXIBLE ET PROCÉDÉ POUR SON UTILISATION

(43) Date of publication of application: 20.05.2015
(73) Proprietor: Lexogen GmbH, 1030 Wien (AT)
(72) Inventor: REDA, Torsten, 1140 Wien (AT); HOLLÄNDER, Igor, 1220 Wien (AT)
(74) Representative: Wildhack & Jellinek
(86) International application number: PCT/AT2012/000182
(87) International publication number: WO 2014/008518

(56) References cited:
- EP-A1- 1 415 710
- MAXIMILIAN FOCKE ET AL: "Lab-on-a-Foil: microfluidics on thin and flexible films", LAB ON A CHIP, vol. 10, no. 11, 1 January 2010 (2010-01-01), page 1365, XP055046349, ISSN: 1473-0197, DOI: 10.1039/c001195a

## Description

### Field of invention

The present invention relates to a sensor carrier and pertains to the design, assembly and use of functionalized surfaces. In particular the invention makes use of flexible films which contain immobilized and surface exposed sensor biomolecules for the use in biosensor array applications. Such biosensor arrays allow multiplex hybridization and selective amplification studies in molecular biology. Consequently, the invention facilitates Lab-on-a Chip or Lab-on-a-Foil solutions built on thin flexible films.

### Background of the invention

### Nucleic acid amplification, surface confined reactions and biosensor arrays

The term biosensor is used for devices which either detect biomolecules or use biomolecules to detect different organic or inorganic analytes. Biosensors are used in a wide spectrum from simple analytical devices targeting one or very few compounds, which are relevant for example in medical diagnostics, examples are hCG, sugar, ethanol to name a few, to complex combinatorial screening platforms which are important for life sciences research. All of them use specific reactions between the sensor compound and the analytes of interest. Such reaction should be converted into measurable signals. Numerous sensor applications are carried out on complex analyte mixtures, e.g. RNA, DNA or polypeptides. The challenge herein is that such analyses target many different molecules and therefore it is required to build large arrays to detect and distinguish those high numbers. Miniaturized sensor arrays have the advantage of limiting the amount of a precious sample.

Nucleic acid sensor arrays can be built with a number of individual oligonucleic acid probes where in each spatial location just one kind of probe is located. Such oligonucleic acid probes are small linear polymers which consist typically of 10 to 50 nucleic acid monomers. The probes should be covalently bound when they engage in stringent washing steps. Hybridization assays can be carried out to detect the annealing of target sequences in so called DNA micro arrays. Furthermore, once a target sequence and probe have hybridized and formed a DNA double strand it can be used to start amplification reactions by polymerases to synthesize complementary copies of the target sequence. With the appropriate opposite primer sequences polymer chain reactions (PCR) can be carried out [Saiki, 1985]. Here, the specific sequences of the first and second primer are used to amplify exponentially the entire or partial target sequence. Those amplification products can be used to generate the detection signal either by labeling during the formation by e.g. fluorescence tags, or through subsequent analysis steps, e.g. transferring the product onto a chromatography gel. Primarily, PCR is a fluid phase reaction in which the two primers, target molecules, dNTPs, polymerases and ions need to react. Such reaction of multiple components makes it inherent difficult to be carried on solid supports. Both primers are immobilized in close proximity, which means in molecular dimensions. One solution to the problem has been presented by grafting surfaces with mixed primers. The immobilization of primers, their accessibility, and subsequent use in PCR have been described as solid phase PCR (SP-PCR), asymmetric SP-PCR, bridge PCR and ESP-PCR [Adessi, 2000; Kahn, 2008]. They are mechanistically limited with respect to amplification efficiency and solid support primer involvement a bridge-PCR process. The PCR on such single solid supports shows low efficiencies of 0.15 compared to values close to 1 in fluid phase systems. Reasons are the two dimensional geometric growth of seeded islands, and the unidirectional perpendicular to surface diffusing reaction compounds.

Alternatively, two different primers can be presented by two different surfaces. Opposite and each other facing surfaces and the imposed directionality are inherently consistent with the working principle of the classical PCR scheme in which two primers bind onto opposite locations in or at the end of a target molecules, and in which the direction of the polymerase catalyzed amplification reaction proceeds in opposite direction. Because the probes of the different surfaces need to interact with the same target molecule it is necessary to bring both surfaces into submicron proximity. The primers should be immobilized either after or before the aligning of the two surfaces. The immobilization afterwards can be achieved by electrochemical stripping as published in WO/2010/104479, which is a long multistep process of limited applicability. Otherwise, the spatial resolution and geometry of two opposite surfaces makes conventional primer deposition methods like spotting and piezo-electric printing which produces feature limits of more than 50 µm, photolithographic deprotection which reaches feature sizes of below 10 µm, or photoresist lithography, µWP, µCP/µFN which enables feature sizes of below 1 µm, absolutely unsuitable. If the individual surfaces become modified before the assembly of the two-surface structure, any of the deposition methods mentioned above can be used. However, the challenge is of how to align two surfaces into submicron proximity. The two solid surfaces cannot touch entirely across large areas and should leave access to the fluid phase. It is possible to realize such assemblies in small scales with liquid media atomic force microscopes (AFM), scanning tunneling microscopes (STM) or electrochemical scanning microscopes (ESCM) which are able to control the distance of two surfaces in molecular dimensions. Multi-tip STMs are sophisticated instruments built for structural analysis. Such techniques are not suitable for the design of complex two surface sensor arrays.

EP 1 415 710 shows molecular arrays with a reflective and a transparent layer enables the interrogation of arrays by suitable scanner that can read the location and intensity of fluorescence.

One solution to the problem has been found in the design of impedimetric sensor arrays which contain numerous dual solid phase nano-gap junctions. Here, the main structural requirements are that small electrodes are electrically insulated with an ultrathin film which is functionalized with sensor molecules, and that those electrodes approach each other to said submicron distances. Those distances are determined by spacers, which are integrated into the individual surface structures as published in [PCT/AT2012/000043]. Such spacers are made during the multistep etching process and controlled by photolithography, the etching conditions and metal track depositions. Because of the close proximity of their surfaces those structures have the disadvantage that any fluid exchange is dramatically slowed down and that it is inherently difficult to design and execute the microfluidics efficiently. This matter constitutes a significant problem. Volumes of the fluidic phase inside the sensor array cannot be varied.

### Flexible Reaction Chambers, Flexible Biosensors and Lab-on-a-Foil devices

Flexible biosensors are well known because many analytical test strips for glucose etc. are built on cheap, thin carrier substrates. Such test strips can be produced on fibrous materials like paper or thin polymer films or foils (foils being semi-finished parts of any material which is thinner than 500 µm).

Fluidic systems have been developed which use the properties of flexible foils to design reaction cassettes and integrated microfluidic devices in which fluid transport can easily be achieved in small volumes [US5863502A1]. Such Lab-on-a-Foil systems are characterized by low material consumption of foils, films, sheets, laminates or tapes, low cost materials, and cost-effective high volume fabrication processes.

Based on such integrated foil approach, the Fraunhofer Institute for Modulare Festkörper-Technologien EMFT (Munich, DE) developed a polymer microfluidic D-dimer biosensor system. D-dimer is a molecular marker for the presence of thromboembolytic events. The biosensor measures impedance changes with interdigitated microelectrodes which are caused by immunological binding of D-dimer to specific recombinant antibodies. The antibodies are covalently immobilised on interdigitated electrodes which are located on a single surface. The microfluidic cartridge consists of microfluidic channels which have been crafted in polycarbonate and which are joined to electrodes on a polyethylenenaphthalate foil.

Thin flexible foils have enabled in addition to utilize various features like low thermal resistance for efficient thermocycling. This is of particular use for PCR which is a popular method to amplify specific genetic sequences. In conventional thermocycling devices one PCR cycle takes approximately 60 to 180 sec. Motorola Inc. had developed an integrated laminate-based polycarbonate foil device for DNA amplification and subsequent hybridization [Liu, 2002]. Cooling and heating were accomplished with a Peltier element reaching fast heating rates of 7.9°C.s⁻¹ and cooling rates of up to 4.6°C.s⁻¹. Systems with thinner foils can reach even faster cycling speeds of up to 88 seconds per cycle which enables 40 amplification cycles in just 30 minutes [Jia, 2007]. Another approaches employed circular shaped foils disk for real-time PCR experiments [Focke, 2010], so-called array tapes for PCR-based assays to detect single nucleotide polymorphism. The array tape contains embossed microwells on continuous tape which passes through different stations of pipetting, drying, sealing etc. [US 6878345].

All examples utilize individual features of flexible foils. Examples have been given where foil devices are used in PCR applications, hybridization and binding reactions with single surface bound sensor compounds.

### Objective of the invention

The main objective was to overcome the above mentioned drawbacks in detection of molecules by finding a solution for biosensor arrays where in molecular dimensions reactions between three compounds - a class of first sensor molecules, a class of second sensor molecules and analyte molecules - are facilitated, wherein the first and second class of sensor molecules are separated, so that a predefined gap is formed between them, and a first portion of specific analyte molecules binds to the first sensor molecules and second portions of specific analyte molecules binds to the second sensor molecules.

Both portions of the analyte molecules should be able to bind simultaneously to a first or a second sensor molecule. These requirements imply that the sensor molecules are separated only by molecular distances of less than one micrometer which is determined by the length of the analyte molecules. The two solid phases and the one fluid phase form a 3-phase region which needs to be maximized in size to increase the sensor functionality. In addition, the fluxes and exchange rates of the analyte molecules between the inner volume phase and the 3-phase region are maximized.

### Summary of the invention

The invention relates to a sensor carrier comprising a flexible film comprising a plurality of functionalized sensor elements each comprising a functional layer, wherein the functional layers are located on the same surface of the film within a window area, being preferably arranged in the centre of the film, wherein a region of each of the functional layers of the sensor elements is functionalized with one or more sensor compounds.

The sensor carrier according to the invention enables to assemble and to form a sensor carrier with flexible films which are functionalized on their respective surfaces. In particular the invention relates to flexible films which contain immobilized surface with exposed sensor biomolecules in dedicated spatial locations. The sensor molecules such as biomolecules are able to react with analyte molecules of a solution. Specific reactions, like the hybridizations of polynucleic acids with shorter oligonucleic acids, lead to signals which are related to the spatial location and quantity of the immobilized sensor molecules. By these means a large number of different reactions can be carried out simultaneously on the surface of a volume and related to the presence and concentration of molecules in the solution. The main advantage of the present invention is the flexibility of the functionalized carrier substrate which enhances the functionality of the sensor and provides additional novel features in contrast to the use of inflexible and stiff carrier substrates.
First sensor molecules are arranged on the first sensor carrier and second sensor molecules are arranged on a second sensor carrier. As to the flexibility of the sensor carriers, a central volume for the analyte can be formed between the window areas of two sensor carriers. Consequently, a reaction as to the objective of the invention is facilitated by the invention.

The flexibility of the film allows movements of the window area relative to the surrounding area.

According to a preferred aspect of the invention it is provided, that the film material has a flexural modulus from 0.5 GPa to 2 GPa, preferably from 0.75 GPa to 1.25 GPa, and/or wherein the thickness of the film is between 10 µm to 300 µm, in particular between 100µm to 200 µm, and/or wherein the film is composed of a polymer, such as polyethylene, polyethylene terephtalate (PET), polyimide, polyethylene naphthalate, polycarbonate, polyether sulphone, polyarylate and/or mixtures thereof

The thickness of the volume between the sensor carriers, i.e. the distance between the sensor carriers, may be modified easily by the application of relatively low external forces, e.g. by tips applied on the surface of the sensor carriers opposing the central volume, without damaging of the sensor carriers.

For the same reason, it can be alternatively or additionally provided, that the flexibility and elasticity of the material of the film, and/or the flexibility and elasticity of the material of the functional layers, and/or the size and/or shape of the window area, are chosen so that, when the boundary of the window area is fixed in direction normal to the plane of the film, and the window area is deflected and/or deformed, out of the plane of the film in the direction normal to the plane of the film, wherein at least one part of the surface is pushed by 10 to 500 µm, preferably by 20 to 200 µm, into the direction normal to the plane of the film, the film is deflected elastically, in particular without being damaged.

In order to have additional visual feedback of the reaction and to be able to apply radiation of light to the analyte molecules, it can be provided, that at least the window area of the film is transparent.

To obtain a sensor carrier which enables a preferable exchange rate of the analyte molecules and a favourable accumulation of analyte molecules to the sensor carrier, it can be provided, that one or more spacers, preferably each having the form of a protrusion, are arranged on the surface of the sensor carrier on the side of the functional layers, wherein the spacers are elevated of the surface of the sensor carrier by 0.05 µm to 1 µm, preferably by 0.2 µm to 0.5 µm.

To obtain sensors for the detection of biomolecules, it can be provided, that the sensor compounds contain oligonucleotides binding to binding sites of analyte molecules, preferably organic polymers or DNA or RNA molecules.

To obtain a sensor carrier whose single sensors can be easily addressed electronically, a sensor carrier can be provided comprising a plurality of electrical conductors, preferably comprising at least one line shaped portion, wherein each of the conductors is assigned to one sensor element, and each conductor, preferably the line shaped portion thereof, is located underneath the functional layer of the respective sensor element in or on the film, and/or wherein the sensor carrier preferably comprises an electrical connector, which is arranged on the film, the electrical connector comprising a number of connector pads, each of the connector pads being connected to one of the conductors, and/or wherein the conductors in or on the film contain or consist of nickel, copper, gold, carbon, indium tin oxide, semiconductors or any other similar conducting material.

To obtain an easily producible sensor carrier, it can be provided, that the film has a rectangular shape, wherein the conductors are arranged in parallel in at least the window area of the film, and wherein at least one portion of each sensor element and/or each conductor, preferably the respective functionalized region of the sensor element, is inclined to the edges of the film, preferably by an angle of 40° to 50°, especially 45°.

The invention further relates to a sensor arrangement comprising a sensor carrier according to the invention, a gasket comprising a central opening, and a second film with a plurality of functionalized sensor elements each being formed by a functional layer, wherein the functional layers are located on the same surface of the second film within a window area, wherein a region of each of the functional layers of the sensor elements is functionalized, and wherein one or more sensor compounds are arranged or located in the respective functionalized regions of the sensor elements, said second film preferably having a plurality of line shaped electrical conductors and one electrical connector and/or with spacers arranged on its surface, wherein the gasket is arranged between the sensor carrier and the second film, so that a chamber is formed between the sensor carrier and the second film, and the chamber is surrounded by the inner edge of the gasket.
With such an arrangement a fully functional sensor, that facilitates the detection of molecules, can be obtained. This arrangement has a preferable design for biosensor arrays where molecular dimensions reactions between three compounds - a class of first sensor molecules, a class of second sensor molecules and analyte molecules - is facilitated. The volume between the sensor carrier and the second film can be easily adopted to the analyte molecules.

A simple arrangement which comprises only one flexible sensor carrier is provided, wherein material of the second film has a flexural modulus higher than 5 GPa. Such a second film may also have the same features as the sensor carrier according to the invention, however the second film need not necessarily have the feature of a high flexibility.

In order to obtain a sensor arrangement with a high density of sensors, it a sensor arrangement can be provided, comprising two sensor carriers according to the invention and a gasket comprising a central opening, wherein the gasket is arranged between the two sensor carriers, so that a chamber is formed between the two sensor carriers and surrounded by the inner edge of the gasket, and wherein the respective surfaces of the sensor carriers, on which the sensor elements are arranged, are facing each other and each sensor element and/or conductor of one of the sensor carriers opposes or faces at least one, preferably each, sensor element and/or conductor of the opposite sensor carrier.

To easily adjust the thickness of the volume by applying pressure with one single tip on the flexible sensor carrier, it can be provided, that particles or beads are arranged between the sensitive opposing surfaces of the sensor carriers within the chamber, wherein the particles or beads are preferably included or dispersed in the analyte fluid containing analyte molecules to by analyzed, wherein the minimum distance between the sensor surfaces of the two films is defined by the diameter of the particles or beads.

In order to obtain a sensor that can be easily filled with an analyte fluid, the sensor arrangement may have an inlet and an outlet leading through one or both of the sensor carriers and/or in the gasket, leading from the outside to the chamber and enabling fluid to stream into the chamber or out of the chamber through the inlet and the outlet.

Furthermore, the invention relates to a cartridge for holding an arrangement according to the invention. Such cartridge comprises an arrangement according to the invention and at least one, preferably two, holder elements wherein the gasket and the sensor carriers or one sensor carrier and one film are fixed and compressed by the holder elements so that the chamber is confined by the gasket, and/or the sensor carriers and/or the film, so that the chamber is sealed and impermeable to liquid. Such cartridge eases handling the sensor arrangement.

In order to obtain a cartridge with which a sensor arrangement can be easily applied a force from one side to set the distance between the films and thickness of the volume and heat from the other side to set the temperature of the fluid within the volume and the temperature of the sensor carriers, it can be provided, that the holder element has at least one opening, wherein the opening is covered by one part of one of the sensor carriers, preferably by the window area, said part at least partially confining the chamber.

To hold a sensor arrangement which enables a preferable exchange rate of the analyte molecules and a favourable accumulation of analyte molecules to the sensor carrier, it can be provided, that the holder element has two opposing openings, wherein the first opening is covered by one portion of one of the sensor carriers, which is preferably the window area of said sensor carrier, and wherein the second opening is covered by one portion of the other sensor carrier or film, wherein the second opening is preferably covered by the window area of the other sensor carrier or film, said portions of the sensor carriers or film at least partially confining the chamber.

In order to obtain a cartridge with which a sensor arrangement can be easily applied a force to set the distance between the films and the thickness of the volume, it can be provided, that the holder element further comprises at least two channels, wherein the first channel connects the inlet with the outer surface of the holder element and the second channel connects the outlet with the outer surface of the holder element.

To avoid the sensor carriers or films of the sensor arrangement from shear movement, it can be provided, that the cartridge comprises two holder elements, wherein
a) the sensor carriers and the gasket, or
b) the sensor carrier and one film and the gasket,
are fixed between the holder elements,
wherein the holder elements preferably comprise protrusions, particularly positioning pins, and holes, matching each other when the holder elements are assembled, and
wherein the sensor carriers, the film and/or the gasket have positioning holes being penetrated by at least some of the protrusions.

To seal the chamber, it can be provided, that the cartridge comprises two holder elements, the holder elements compressing the arrangement to seal the chamber.

It is a further objective to determine the amount of a specific analyte compounds in an analyte fluid, where only analyte compounds of a specific molecular length can be determined.

The invention solves this objective with a method according to the invention. The invention relates to a method for the measurement of a concentration of the analyte compound in an analyte fluid, preferably with two sensor carriers or a sensor arrangement or a cartridge according to one of the preceding claims,
a) wherein an elastically flexible first film and a, preferably elastically flexible, second film are provided, a first sensor compound being arranged on a region of the first film and a second sensor compound being arranged on a region of the second film, the sensor compounds being arranged on opposing sensor surfaces of the films facing each other,
b) wherein the first sensor compound binds or adheres to a first portion of the molecule of the analyte compound and the second sensor compound binds or adheres to a second portion of the molecule of the analyte compound, and
c) wherein the analyte fluid is arranged in a chamber formed between and surrounded by the two films,
d) wherein the first film and the second film are elastically deflected or deformed in a manner, that the distance between the opposing surfaces of the films facing each other is reduced and equals or is smaller than the distance between the first portion and the second portion of the molecule of the analyte compound,
e) so that a sensor is formed between the region of the first film on which the first sensor compound is arranged and the region of the second film on which the second sensor compound is arranged, where said regions are arranged to face each other, and
f) wherein the amount of molecules bound or adhered to the first and second sensor compounds is measured.

In order to determine the distance between the films, it can be provided, that particles or beads of a given diameter are added to the analyte fluid, wherein the particles or beads are filled into the chamber between the two films, so that the minimum distance between the sensor surfaces of the two films is defined by the diameter of the particles.

In order to automatically determine the amount of the analyte compound in the analyte fluid, it can be provided, that the capacitance or impedance between the electrical conductors in the regions of the first film and the second film on which sensor compounds are arranged is measured, and said capacitance or impedance indicating the amount of the analyte compound in the analyte fluid.

To determine the amount of a plurality of different analyte compounds in parallel, with two sensor carriers according to the invention are used, each of which comprises one film or with an arrangement according the invention or a cartridge according to the imvention comprising the films, wherein a plurality of sensors is formed between the regions of the first film on which the first sensor compound or plurality thereof is arranged and the regions of the second film on which the second sensor compound or plurality thereof is arranged, where said regions are arranged to face each other, and wherein the concentration of analyte compounds located or deposited between two facing regions of a sensor is determined separately for each sensor.

In order to control the amount of analyte fluid in the chamber, it can be provided, that before setting the distance of the opposing films the distance of the films is increased in order to increase the amount of fluid within the chamber and/or the distance of the films is regulated to adjust the internal volume of the chamber in order to control the amount of analyte fluid accommodated in the chamber.

In order to increase or decrease the velocity of the analyte compounds in the analyte fluid and to remove partially bound analyte compounds from the sensor compounds of the films, it can be provided, that the distance between the films is, preferably repeatedly, changed before or during the measurement so that the analyte fluid in the chamber is mechanically agitated, so that by adjusting the flow in chamber a specific shear force distribution profile is achieved in the chamber.

In order to fill or empty the chamber before or after measurement, it can be provided, that the distance between the films is changed in order to pump fluid into the chamber or out of the chamber.

### Brief Description of the Drawings

Some examples of the invention are illustrated in non-limiting manner with reference to the following drawings.

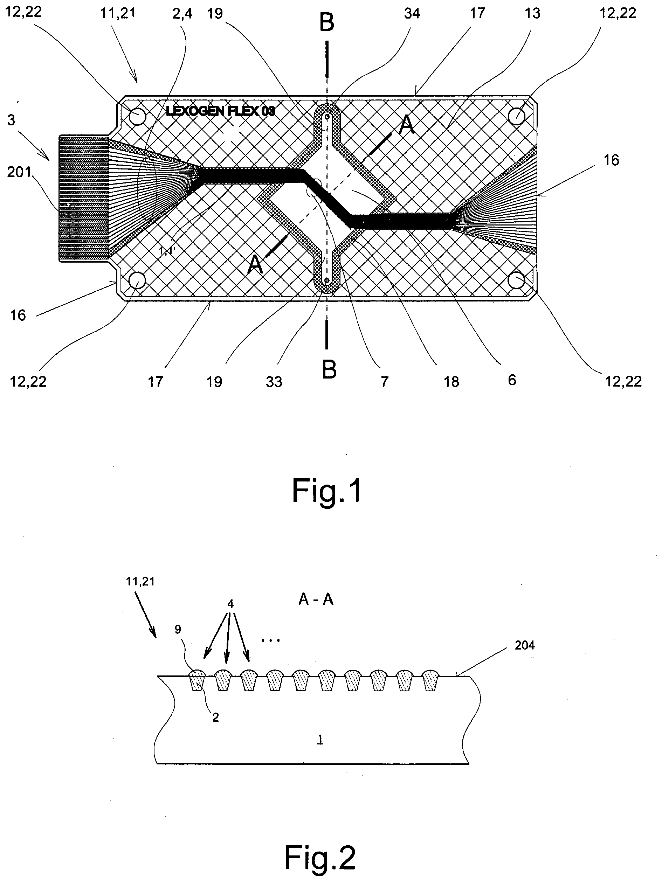

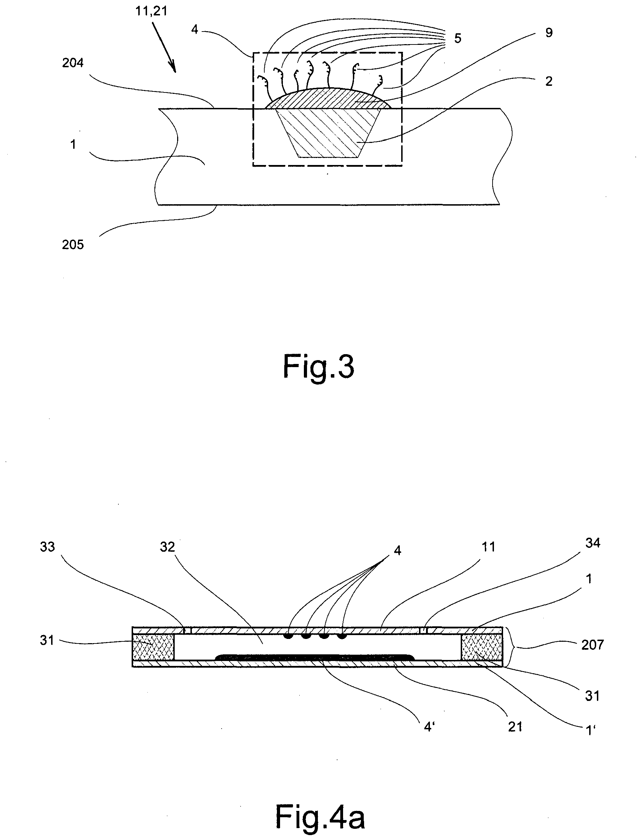

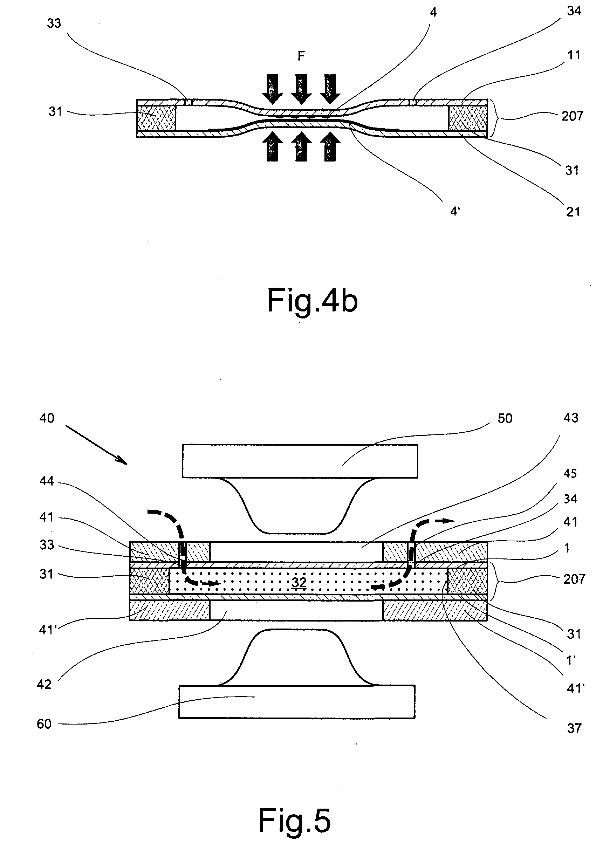

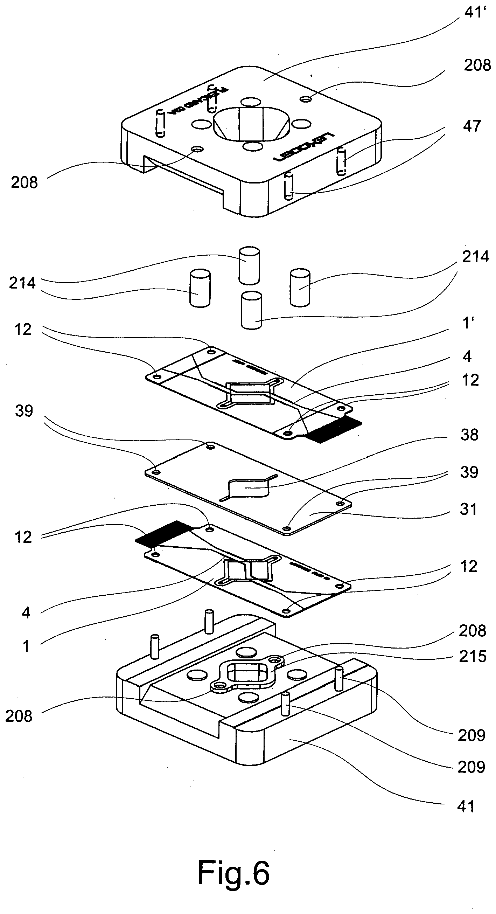

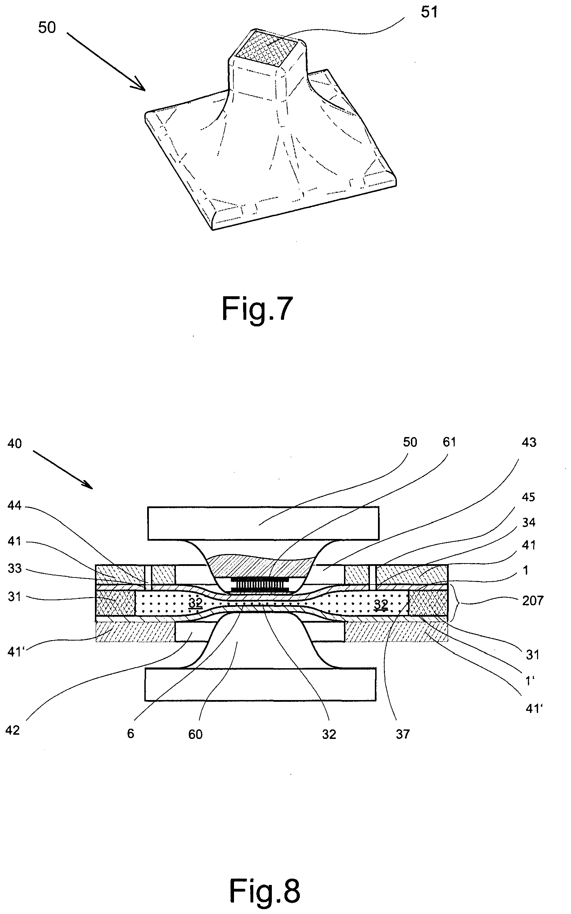

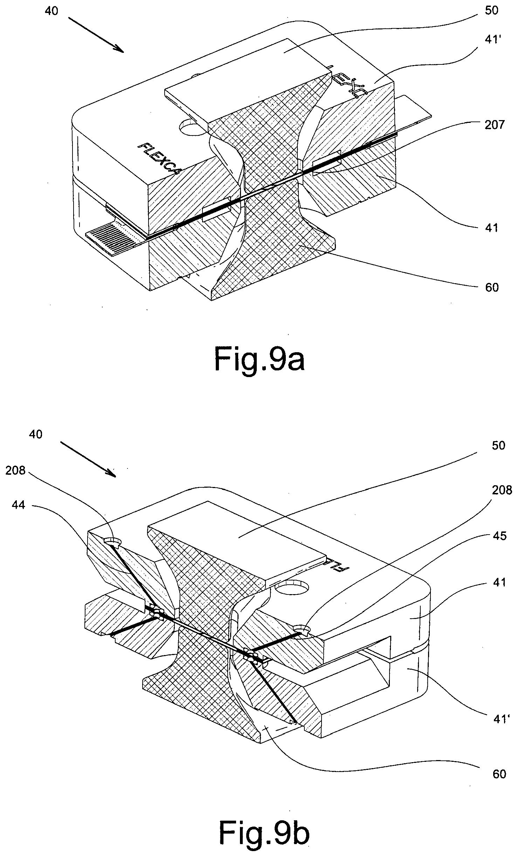

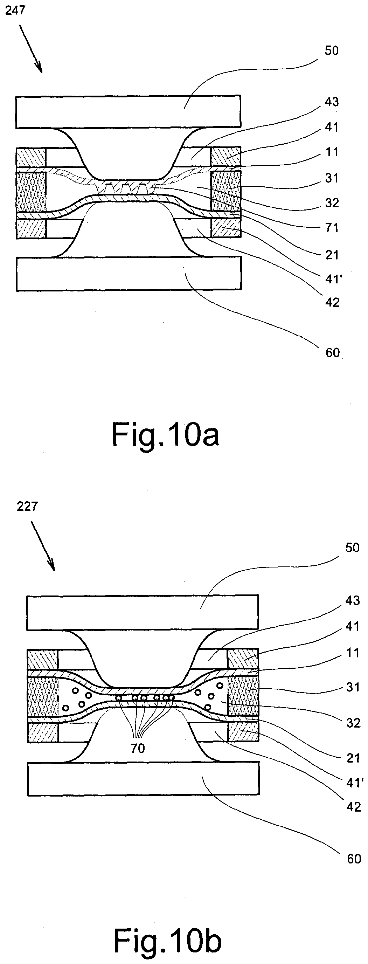

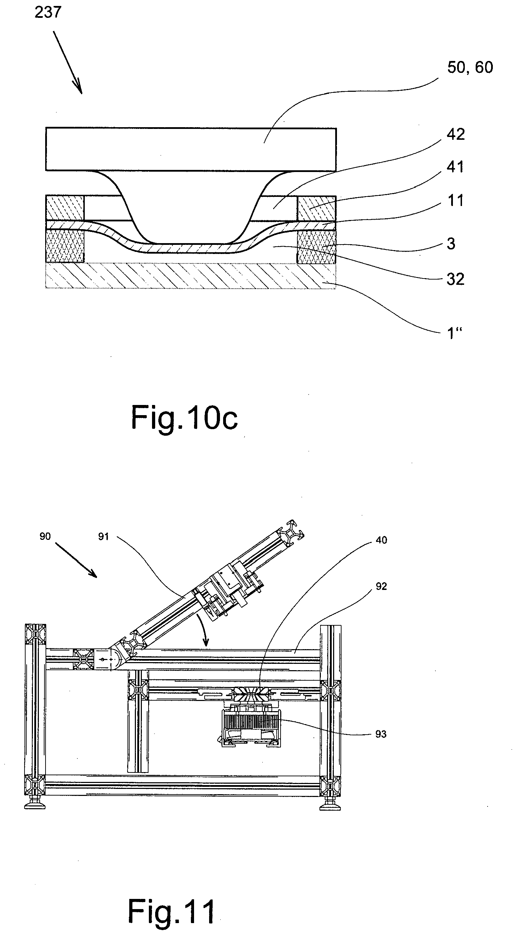

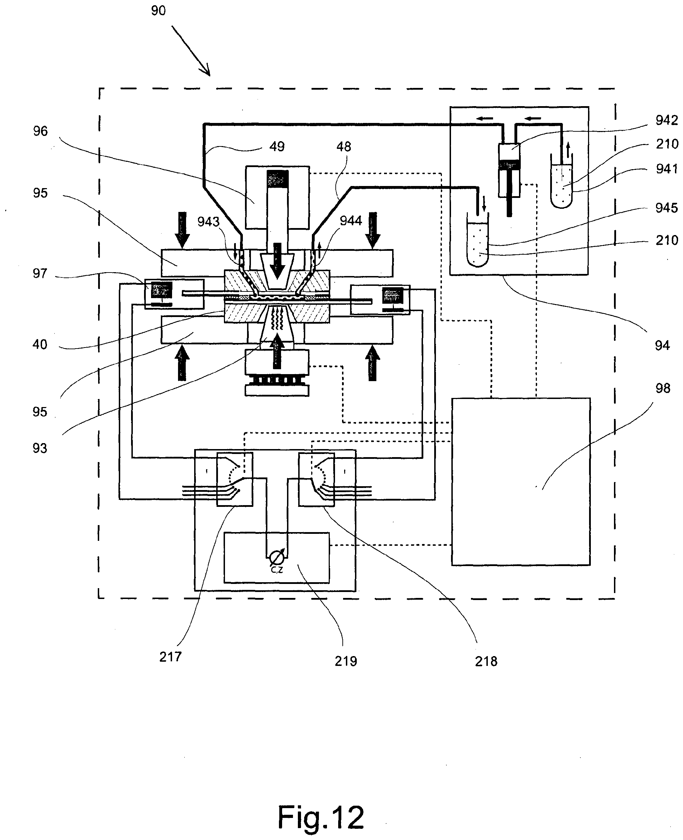

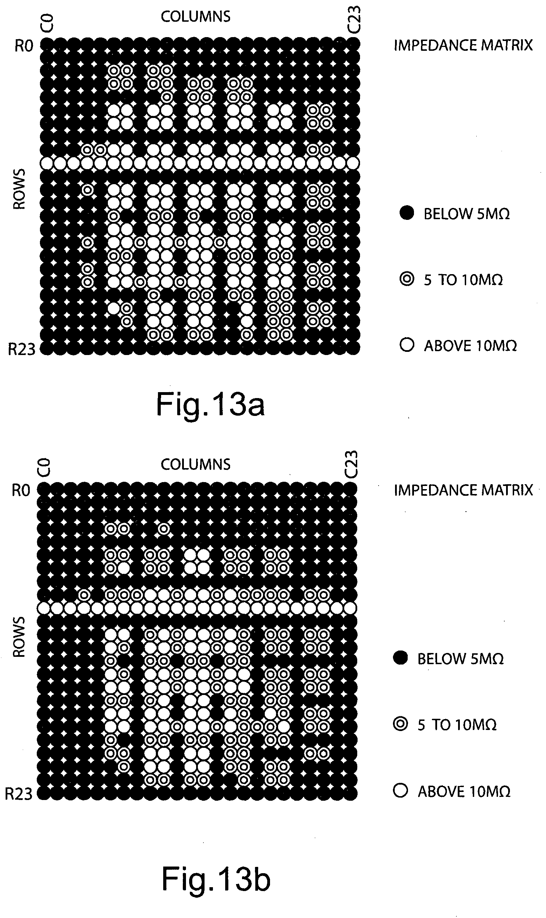

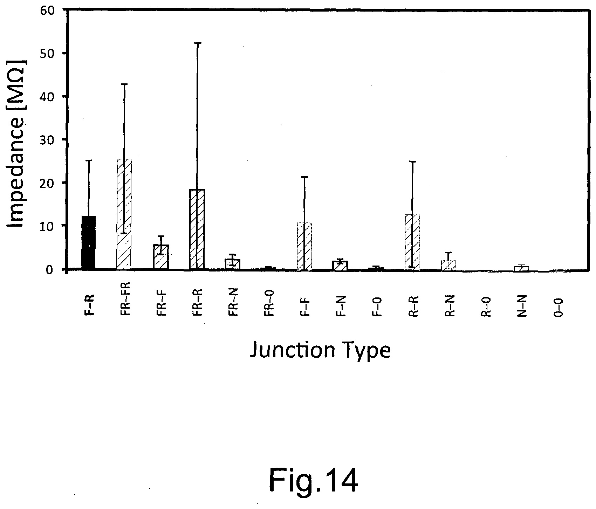

**Fig. 1** presents the drawing of an example of a sensor carrier according to the invention in top view. **Fig. 2** illustrates a partial section of the sensor element of **Fig. 1** of plane A-A. **Fig. 3** highlights a detail of **Fig. 2** with a sensor element with functional coating. **Fig. 4a** illustrates a part of the section of plane A-A of an arrangement comprising two sensor carriers as shown in **Fig. 1** without external application of pressure, the unloaded state. **Fig. 4b** illustrates the arrangement of **Fig. 4a** where external pressure F is applied via a tip, loaded state. **Fig. 5** shows a cross section of a sensor cartridge comprising the arrangement of **Fig. 4a** and **4b****.** **Fig. 6** displays the cartridge of **Fig. 5** in exploded view. **Fig. 7** shows a heating tip. **Fig. 8** illustrates the cartridge with an arrangement of **Fig. 5** where external pressure is applied via a tip. **Fig. 9a** and **9b** show sectional views of a cartridge of **Fig. 6** being equipped with two tips for heating and application of pressure. **Fig. 10a** shows a sensor arrangement in the loaded state having a film-gasket-film sandwich structure, where one of the sensor carriers features spacers on its surface. **Fig. 10b** illustrates a sensor arrangement in the loaded state having a film-gasket-film sandwich structure, where the films of the sensor carriers are kept separated by particles. **Fig. 10c** shows an arrangement having a film-gasket-film sandwich structure, where the flexural rigidity of one of the films is substantially higher than the flexural rigidity of the other film. **Fig. 11** illustrates a section view of reader instrument with a cartridge placed in the mechanical adaptor with a clamping mechanism formed by one upper and one lower arm, and the thermocycling adaptor. **Fig. 12** schematically depicts an overview scheme of the reader instrument. **Fig. 13a** shows the impedimetric scanning of an electrode junction array before PCR with a target DNA sequence present in solution. **Fig. 13b** shows the impedimetric scanning of an electrode junction array after 40 PCR cycles under otherwise the same conditions. The mean and standard deviation of the differences in the impedance modulus of identical junctions with respect to their functionalization are shown in **Fig. 14****.** The functionalizations are encoded with F, forward primer; R, reverse primer; N, non matching primer; and 0, no primer.

### Detailed description of preferred embodiments

A sensor carrier **11** according to a **first example** of the invention is depicted in **Fig. 1****.** The sensor carrier **11** comprises a flexible film **1** with twenty four functionalized sensor elements **4.** The base film **1** of the sensor carrier is made of a heat stabilized polyethylene terephthalate (PET), such as MELINEX ST504 (DuPont Teijin Films, USA).

Typically, the film **1** can be made of any material which is not permeable for the constituents of the solution used in the reaction, is compatible with, i.e. inert with respect to, the reaction, and withstands the temperature changes required for PCR thermocycling under mechanical load without permanent deformations. Optimally, the material has a good thermal conductance, so the rapid temperature changes of the analyte in the chamber **32,** i.e. on the sensor side **204, 204'** of the film **1,** can be achieved when the film is cooled or heated on its non-sensor side **205, 205'.** It is of advantage to use a transparent material if optical readout with light excitation and emission detection should be carried out through the film. From the point of view of the raw biosensor production technology, materials such as Polyimide (PI), Polyethylene Naphthlate (PEN), Polyethylene Terephthalate (PET), Polycarbonate (PC), Polyether Sulphone (PES), Polyarylate and other can be used.

The film **1** is typically produced by technologies used for flexible circuit boards (FCB) or similar, combined with chemical technologies used for coating the film surface with insulating layers and with layers exposing the immobilized reaction partners, referred to as 5'-end and 3'-end primers, or also referred to as forward (F) and reverse (R) primers. It is particularly suitable to produce the films **1** using the ultra-high resolution flexible circuit boards technology. Such technologies are known in the art and have been disclosed, e.g., in the document US 6,284,072. The advantages offered by these technologies include very small structures, down to 3 µm track width and 5 µm gap width, and nearly planar surfaces. Unlike the standard FCB manufacturing process, resulting in polymer substrates with electrical conductors **2** with typical height of 10 to 50 µm over the base surface, the said ultra-high resolution FCB technology produces electrical conductors **2** formed by metallic tracks which are embedded in the substrate, so that resulting surface is planar with roughness 5 to 15 nm (rms). The tracks of the electrical conductors **2** are typically made of nickel with gold coating.

The material of the film **1** has an upper temperature limit of 140°C and good optical and mechanical properties. The Young's modulus of the material of the film **1** is 2.0 to 2.7 GPa. The ultimate tensile strength of the material of the film **1** is 55 MPa. Poisson's ratio of the material of the film **1** is between 0.37 to 0.44. Thermal conductivity of the material is 0.15 to 0.40 W.m⁻¹.K⁻¹. Thickness of the film **1** is 125 µm. The flexural modulus of the material of the film **1** is 1 GPa. The flexural modulus of the film can for instance be determined by the ASTM D 790 standard.

As shown in **Fig. 2** the film **1** has recesses in which electrical conductors **2** are embedded to form conductive tracks. These tracks or conductors **2** are manufactured using specific technology for ultra-high density flexible circuit boards. The electrical conductors **2** are made of nickel with a thickness of 5.4 µm, which are coated with a gold layer of 0.1 to 0.2 µm thickness. The surface of the electrical conductors **2** is almost isoplanar to the surrounding PET surface, with a height difference of up to 100 nm. The overall thickness of the film **1** including the layer with embedded electrical conductors **2** is 145 µm.

In the first example of the invention, the film is rectangular with an outline of 65x25 mm. The outer dimensions of the film allow to position the film into a standard microarray scanner and to scan it similarly to a microarray on a 25x75 mm glass carrier. It is also a convenient size for inspection using optical microscopy. The electrical conductors **2** of the sensor elements **4** are connected to an electrical connector **3** comprising a plurality of connector pads **201,** wherein typically, each sensor element **4** is connected to a single connector pad **201.** The electrical connector **3** is located typically in the marginal area of the sensor carrier **11, 21.** On both shorter edges **16,** the film **1** comprises electrical connectors **3** which are in this very example formed as tongues having a width of 15.5 mm and a length of 7 mm. The form and arrangement of electrical connectors **3** and connector pads **201** match a standard industrial 30 way, 0.5 mm pitch FCB connector. This electrical connector **3** connects the conductors **2** of the sensor carrier **11, 21** with external electronics circuits **219** located in the readout instrument **90 (****Fig. 12****).** Note that in **Fig. 1****,** the electrical connector **3** is only shown on one shorter edge **16,** as the electrical connector **3** on the other shorter edge **16** is typically cut off before application (see below).

The film **1** has twenty four electrical conductors **2** in the form of partly linear tracks, leading from one electrical connector **3** located on one short edge **16** of the film **1,** via a window area **6** of the film **1,** to the other electrical connector **3** on the opposite short edge **16** of the film **1.** In the window area **6** the electrical conductors **2** are linear and parallel to each other. The conductors **2** are 25 µm wide, and aligned in a 50 µm pitch.

In the window area **6** the angle between the short edges **16** and the long edges **17** of the film **1** and the electrical conductors **2** is 45°.

The electrical conductors **2** are connected to the connector pads **201** in the center of the 30-way connectors **3** located on both sides of the film **1.** The six remaining outer connector pads **201** are shorted and electrically connected to a shielding **13.**

The film **1** of the sensor carriers **11, 21** has four positioning holes **12** with a diameter of **2** mm each in the four corners, and two fluidic openings **33, 34** with a diameter of 0.5 mm, forming an inlet **33** and an outlet **34** of a sensor arrangement **207.**

The window area **6** of the film **1** has a squared shape which is surrounded by a diamond shaped shielding element **18.** The edges of the shielding element **18** and the edges of the film **1** are rotated by 45°. Two corners of the diamond shaped shielding element **18** which are near the longer edges **17** surround two channels **19,** where each of the channels **19** contains one fluidic opening **33, 34.**

The remaining area of the film **1** outside the window area **6** which is not covered by the electrical conductors **2,** connector pads **3** or positioning holes **12** is covered with a hatching pattern shielding **13** consisting of further electrical conductors. The conductors of that hatching pattern shielding **13** are arranged for technological reasons to avoid curling of the film **1.** This hatching pattern shielding **13** is connected to the shielding element **18** of the window area **6** and the six unused connector pads **15** of the electrical connectors **3.**

Immediately after production, the film **1** has two tongue shaped connectors **3** on which connector pads **201** are arranged.

For quality control purposes, the electrical conductors **2** of the sensor elements **4** lead from the electrical connector **3** on one short edge of the film **1** via the window area **6** of the film **1** to another secondary electrical conductor **3** on the opposing short edge of the film **1,** allowing to check the integrity of the electrical conductors **2.** In order to quality control the fabrication of the conductors **2,** both electrical connectors **3** are attached to an external electrical circuit, wherein the resistance of each electrical conductor **2** can be measured. Possible short connections between the electrical conductors **2** and/or broken electrical conductors **2** can be detected. A typical break detection method involves connecting the two corresponding connector pads **201, 201'** of one electrical conductor **2** to an electrical circuit and measuring the resistance which should ideally be close to zero Ohm. A typical short detection method consists in measuring the resistance between any neighboring two electrical conductors **2,** which should ideally be infinitely high. After this quality check, one of the tongue-shaped electrical connectors **3** may be cut off.

In a next production step the films **1** of the sensor carriers **11, 21** are functionalized by a specific electrochemical process. **Fig. 3** shows a detailed section view through the sensor carrier **11, 21** with a sensor element **4** comprising a functionalization coating or layer **9** on top of an electrical conductor **2.** It is not necessary that the electrical conductor **2** is fully covered by a functionalization layer **9,** moreover it is possible that only smaller regions **7** located directly above the electrical conductor **2** are functionalized and covered with a functionalization layer **9.** Preferably all the functionalized regions **7** are located in the window area **6** of the sensor carrier **11, 21.**

In order to form a functionalization layer **9,** sensor compounds **5** are immobilized through the functionalization coating **9** which produces the layer **7** of the sensor element **4.** The sensor compounds **5** are exposed on the sensor surface **204** of the sensor carrier **11, 21.**

Each of the electrical conductors **2** may be coated with an individual insulating functionalization layer **9** or coating comprising an individual sensor compound **5.** In this very example, the sensor compound **5** comprises immobilized oligonucleotides which are exposed to the surface of the electrically insulating functionalization layer **9.**

**Fig. 4a** illustrates a sensor arrangement **207** of two sensor carriers **11, 21** as shown above forming a sandwich structure, where a gasket **31** is placed between the sensor carriers **11, 21.** As already mentioned, both sensor carriers **11, 21** have the same dimensions and are made of polyethylene terephthalate (PET). The sensor carriers **11, 21** comprise a flexible film, which can easily be deformed, i.e. bended and flexed by hand. A series of linear sensor elements **4,** i.e. electrical conductors **2,** up to 1000 per film **1,** typically possessing a width of several microns to several tens of microns, are arranged in a parallel pattern on the sensor carriers **11, 21.** In this first example the films contains 24 electrical conductors **2.** Each of the sensor elements **4** comprises an electrical conductor **2** and a functionalized layer **9** or coating. The electrical conductor **2** is covered with the functionalized layer **9** or coating in at least one surface region **7** of the film **1,** which is usually located in the window area **6** of the film **1.** The electrical conductor **2** are realized on the same side of the film **1.**

Typically an arrangement **207** is formed in a sandwich shape comprising the following layers: a first sensor carrier **11,** a gasket **31** being arranged on the sensitive sensor surface **204** of the sensor carrier **11,** the gasket **31** comprising a central opening **38** matching the window area **6** of the first sensor carrier **11,** and a second sensor carrier **21** with the sensitive sensor surface **204** facing towards the gasket **31** and the sensitive sensor surface **204** of the first sensor carrier **11.** The two sensor carriers **11, 21** of the arrangement **207** are geometrically identical, where the functionalized coatings or layers **9** of the single sensor elements **4** may contain different sensor compounds **5.**

In this very example, the arrangement **207** comprises two sensor carriers **11, 21** with identical geometry. The form and size factor of the chip are chosen to be compatible with the standard sample platforms used in molecular biology. This allows using the sensor carriers in combination with different sample preparation and detection/analysis technologies used in this scientific domain, such as microarray spotting, microarray scanning, optical and other microscopy etc.

The gasket **31** of the arrangement **207** is typically made of elastic material and has an outline form and a size corresponding to the film sensor carriers **11, 21,** with exception of the area of the connector pads **201** of the film. The typical thickness of the gasket **31** is from 100 to 250 µm. In this example, the gasket **31** consists of a polytetrafluoroethylene (PTFE) or Teflon film (Polytetra, Germany) with a thickness of 250 µm.

The outer edge of the gasket **31** matches the outline of the sensor carriers **11, 21,** wherein the gasket **31** does not cover the electrical connectors **3.** The gasket **31** has four holes matching the positioning holes **12** of the sensor carriers **11, 21.**

The areas of the connector pads **201** area of both films **1, 1'** stand out of or project from the assembly and are accessible for electrical connection. Thus, all the sensor elements **4** of sensor carriers **11, 21** are electrically accessible for impedance and/or capacitance readout.

The gasket **31** has a central opening **38,** typically of a square or round shape, which covers the window area **6** of the film when the outlines of the film **1** and the gasket **31** are aligned to each other. The central opening **38** of the gasket may be slightly larger with respect to both planar directions than the window area **6** of the film **1.** This opening **38** is arranged so that, when the gasket **31** and the film **1** are aligned, it also covers the two fluidic openings **33, 34** of the film **1.** In this very example, the inner boundary edge of the gasket **31** matches the shielding **18** of the window area **6,** so that consequently the opening **38** of the gasket **31** is connected to the inlet **33** and the outlet **34.**

The gasket **31** also has positioning holes **39,** which, when the gasket **31** and the sensor carriers **11, 21** are aligned, coincide with the positioning holes **12** of the film **1.** The form of the gasket **31** allows the area of the connector pads **201** of the film **1** to protrude from the gasket **31** and remain accessible for an external connector, even when the sensor carrier **11, 21** is arranged in a sensor arrangement **207.**

When the said sandwich arrangement **207** of a first sensor carrier **11,** a gasket **31,** and a second sensor **21** carrier is assembled, a chamber **32** or compartment is formed between the gasket **31** and the sensor carriers **11, 21.** The volume of the chamber **32** is confined by a part of the sensor side surface **204** of the first film **1,** by the part of the sensor side surface **204'** of the second film **1',** and by the side of the inner edge of the gasket **31.**

The window areas **6** of the sensor carriers **11, 21** are exposed to this chamber **32.** The chamber **32** is fluidically connected with the non-sensor surface **205** of the first sensor carrier **11** opposing the sensor surface **204** and the non-sensor surface **205'** opposing the sensor surface **204'** of the second sensor carrier **21** by the fluidic openings **33, 34** in the films **1** of the sensor carriers **11, 21.**

In order to obtain a flow of a fluid through the chamber **32** formed between the sensor carriers **11, 21** and the gasket **31** there is at least one inlet **33** and one outlet **34.** It is possible that both inlet **33** and outlet **34** are located in one of the sensor carriers **11, 21.** It is however also possible that the inlet **33** is located in one and the outlet **34** is located in the other of the sensor carriers **11, 21.**

Usually, only two of the four fluidic openings **33, 34** and **33', 34'** in the films of the first and second sensor carrier **11, 21** are used as fluid inlet **33** and outlet **34.** The remaining two holes are sealed at the non-sensor surface **205** of one of the sensor carriers **11, 21.** Even if the two fluidic openings **33, 34'** are not used, it is of advantage that these fluidic holes are available in the films of both sensor carriers, because there both sensor carriers **11, 21** may be produced by one single unified design.

The film has openings **33, 34** which serve for fluidic connection of the both sides of the film **1.** The reaction fluid is located in the chamber **32** which forms a volume compartment, one wall of which being formed by the sensor surface **204** of the film **1.** The other side of the film **1,** i.e. the non-sensor surface **205** of the film **1,** is connected to a fluidic adaptor **94** of the reader instrument **90** as shown in **Fig. 12** accommodating the film **1.** The fluidic openings **33, 34** are typically positioned near the window area **6** of the film **1,** in a symmetrical pattern. Typically, each film **1** has two fluidic openings **33, 34,** one of the fluidic openings serving as an inlet **33** and one of the fluidic openings **34** serving as an outlet. Since the arrangement **207** of the holes is symmetrical, their function can be freely exchanged.

To help accurate alignment of the films **1** and **1'** and the gasket **31** to each other, the films **1, 1'** and the gasket **31** also have typically four alignment or positioning holes **12, 22, 39** in the four corners of the rectangular shape. These holes **12, 22, 39** serve as positioning aid, and, in the sensor arrangement, are penetrated by protrusions formed by positioning pins **209** fixed in the external holder element **41.**

In order to seal the chamber **32** and to avoid fluid inside the chamber **32** from leaking a further holding force is applied in the region around the circumference of the central opening **38** of the gasket **31.** The applied force is directed normal to the films **1** of the sensor carriers **11, 21.** Such a force can be applied by magnets.

In the initial state of the sensor arrangement **207** as shown in **Fig. 4a** the distance between the sensor side surface **204** of the first film **1** and the sensor side surface **204'** of the second film **1'** is given by the thickness of the gasket **31.** This distance or clearance allows for convenient filling of the fluid into the chamber **32.** Hereby fluid comprising analyte molecules is filled into the chamber **32** via the inlet **33,** while the fluid or gas within the chamber **32** is pushed out of the chamber **32** through the outlet **34.**

In the window area **6** of the film **1,** the sensor elements **4** are arranged in parallel and in such a way that when two identical sensor carriers **11, 21** are brought together and aligned with the electrical conductors **2** and sensor elements **4** sides facing each other, the sensor elements **4** form a mesh, wherein each sensor element **4** of the first sensor carrier **11** faces or opposes each sensor element **4** of the second sensor carrier **22** in at least one portion, referred to as junction. Typically, the films **1, 1'** have a rectangular or similar shape. The sensor element bundle in the window area 6 is arranged at an angle of 45° relative to the sides of the film **1.** Thus, when two such sensor carriers **11, 21** are brought together with the outlines aligned, the sensor elements **4** in the window area of the film **1** of the first sensor carrier **11** cross the sensor elements **4** in the window area 6 of the film **1'** of the second sensor carrier **21** at the right angle, forming a rectangular grid of junctions. The window area **6** is then defined by the square including all the junctions of the rectangular grid, the sides of the square being at an angle of 45° relative to the sides of the film outline rectangle. Typically, the centre of this square coincides with the centre of the rectangular outline of the film **1.**

In **Fig. 4b** the application of pressure to the sensor carriers **11, 21** is shown in detail. The sandwich shaped arrangement **207** is compressed by applying compression force F to the window areas **6** of the sensor carriers **11, 21.** As shown in **Fig. 4b****,** compression force F is applied on both opposing carriers **11, 21.**
Since the elasticity of the material of the films **1, 1'** of the sensor carriers **11, 21** and the thickness of the films **1, 1'** allow for relatively easy elastic deformation with respect to forces and pressures typically applied, the window areas **6** of both sensor carriers **11, 21,** which are not in direct contact with the gasket **31,** can be elastically deflected in direction towards or away from each other, if suitable forces are applied. Due to this deformation, the volume and the thickness of the chamber **32** decreases, i.e. negative deformation, or increases, i.e. positive deformation, respectively. Consequently, the distance between the sensor side surfaces **204, 204'**of the films of the first and the second sensor carrier **11, 21** in the window area **6** can be controlled by the applied forces.

The distance between the sensor side surfaces **204, 204'** of the films **1, 1'** may be regulated by applying force to the fluid within the chamber **32** and thereby deform the films **1, 1'** so that the distance between these films **1, 1'** is increased. In order to decrease the distance between the films **1, 1'** mechanical force can be applied from outside of the sandwich **207** by compressing the window area **6** of the sandwich **207** from both sides by suitable shaped tips **50, 60** as shown in **Fig. 5****.**

Simultaneous binding or hybridization reaction of analyte molecules inside the chamber **32** and electrical impedance and/or capacitance measurements are dependent on the distance between the sensor side surfaces **204, 204'** of the first **1** and the second film **1'.** Therefore setting the distance between the sensor side surfaces **204, 204'** of the films **1, 1'** may be used to adapt the respective sensor arrangement **207** to certain kinds of hybridization reactions for molecules of a given length.

To mechanically hold the sandwich shaped sensor arrangement **207** during manipulation and also to enable a convenient fluidic, electrical, and thermal connection to the reader instrument **90 (****Fig. 12****),** it is of advantage to enclose the said arrangement **207** in a cartridge **40.** An example of such a cartridge **40** is shown in **Fig. 5****.** The cartridge **40** comprises two holder elements **41.** Each holder element **41** has the form and size to carry the sensor arrangement **207** and is convenient for manipulation. In the example shown in **Fig. 5****,** the cartridge **40** has the shape of a rectangular block of about 50x50x20.5 mm made of a transparent polymer, typically polycarbonate (PC).

The arrangement **207** is fixed between two similar holder elements **41, 41'** and aligned with positioning pins **209,** which are attached to one of the holder elements **41, 41',** and passing through the alignment or positioning holes **12, 22, 39** in the first film **1,** in the gasket **31,** and in the second film **1'.** The second holder element **41'** has openings **47** which match the position of the positioning pins **209.** The holder elements **41, 41'** also have a mechanical, electromagnetic, magnetic or other similar clamping mechanism, which allows keeping the two holder elements **41, 41'** with the film-gasket-film sandwich **207** together. Typically, the holder elements **41, 41'** have four permanent magnets **214** fixed and aligned around the central opening **42,** with suitable magnetic polarity, so two similarly assembled holders elements **41, 41'** are held together by magnetic forces.

Each of the holder elements **41, 41'** has one central opening **42, 43** coinciding with the window area **6** of the sensor carriers **11, 21.** Furthermore the holder elements **41** of the cartridge **40** have two fluidic channels **44** and **45** coinciding, on the holder side facing the sensor carriers **11, 21,** with the fluidic openings **33, 34** in the film **1.**

**Fig. 6** shows an explosion view of the cartridge **40** formed by two holder elements **41, 41'** and of the sandwich formed by two sensor carriers **11, 21** and a gasket **31.** The holder element **41** contains positioning pins **209,** which penetrate the positioning holes **12** in the films **1, 1'** of the sensor elements, and the positioning or alignment holes **39** of the gasket **31,** and are inserted into the holes **47** in the opposing holder element **41'.** The cartridge **40** is held together by magnetic forces exerted by permanent magnets **214** fixed in the holder elements **41, 41'.** The sensor carriers **11, 21** and the gasket **31** of the sensor arrangement **207** are compressed by the magnetic forces of the magnets **214,** in particular in the rim area **215.**

The central opening **42** of the holder element **41** allows access to the window area **6** of one of the films **1, 1'** from the film-gasket-film arrangement **207,** so in the full cartridge **40,** the window areas **6** of both films **1, 1'** in the arrangement **207** are accessible from opposite sides.

The holder **41** has fluidic channels **44, 45** which coincide with one end (film end) with the fluidic openings **33, 34** in the films, and with the other end (instrument end) with fluidic connection in the reader instrument **90.** The reaction fluid or analyte fluid **210** can thus be filled from the reader instrument **90 (****Fig. 12****)** through one of these fluidic channels **44, 45** into the chamber **32** of the sandwich **207.** The fluidic channels **44, 45 (****Fig. 9a, 9b****)** may be drilled in the direction normal to the holder side facing the arrangement **207.** Alternatively, they can be made in a more complex manner, to adapt the position of the instrument end to the specific design and requirements of the reader instrument **90.** Both of the two holder elements **41, 41'** may have the fluidic channels **44, 45** which allows for a single unified design of the holder. In this case, the fluidic channels **44, 45** in one of the holder elements **41, 41'** can be blinded or sealed and not used. Alternatively, there might be two different types of holder elements **41, 41',** one with and another without the fluidic channels, wherein a complete cartridge **40** is always assembled using one holder with fluidic channels and one holder without fluidic channels.

On the side of the holder element **41** which is facing the film **1** is a rim feature **215** matching the size and the form of the window area **6** of the film **1,** and in particular, the size and the form of the central opening **38** of the gasket **31.** The rim **215,** typically 1 to 2 mm wide, is made around the shape of the central opening **38** of the gasket **31.** When two holder elements **41, 41'** are assembled to a cartridge **40,** the rim **215** of the holder element **41** matches the rim of the opposite holder element **41',** and they both compress the two films **1, 1'** around the margin of the central opening **38** of the gasket **31.** Thus, when the two holder elements **41, 41'** are additionally compressed by a force applied in the direction normal to the film-gasket-film sandwich **207,** this force allows tight sealing of the chamber **32** in the sandwich **207.**

The connections between the sensor carriers **11, 21** and the fluidic channels **44, 45** in the holder elements **41, 41',** and the connection between the channels **44, 45** and fluidic channels **48, 49,** in the reader instrument **90** are shown in **Fig. 12****.** The connections are sealed by O-rings which are placed in corresponding pockets **208 (****Fig. 9b****).**

To allow for sealing of the fluidic connection between the fluidic channels **44, 45** in the holder element **41** and the fluidic openings **33, 34** in the film **1,** the holder elements **41, 41'** have said pockets **208,** i.e. shallow holes, that are concentric with the location of the fluidic openings **33, 34** in the film **1** and the respective ends of the fluidic channels **44, 45.** If silicone rubber or similar elastic O-rings of suitable size, with a free state height slightly greater than the depth of the pocket, are placed into the pockets **208,** the fluidic connections between the holder elements **41** and the film **1** are sealed. The holder elements **41** can also have similar pockets **208** with O-rings at the instrument side of the fluidic channels **44, 45,** when similar fluidic interface is provided by the reader instrument **90.**

When assembled, the cartridge **40** has dimensions of 50x50x20.5 mm, the last dimension being the sum of the height of two holder elements **41** and the film-gasket-film arrangement **207.** The fluidic channels **44, 45** connect the outer side of the cartridge **40** with the chamber **32** in the arrangement **207.** In this very example, only the fluidic openings **33, 34** of the upper holder element **41** are used. The fluidic connections between the lower sensor carrier **11** and the lower holder element **41'** are blinded and sealed by replacing a sealing O-ring with a matching size silicone disc.

The electrical connectors **3** of the sensor carriers **11, 21** are accessible from outside the cartridge, because the connectors **3** have the shape of tongues protruding from the films **1, 1' of** the sensor carriers **11, 21** and extend beyond the cartridge **40.** Special zero-insertion force connectors may be used to electrically contact the connectors **3** of the sensor carriers **11, 21** of the cartridge to the reader instrument **90 (****Fig. 12****).**

**Fig. 7** illustrates the tip **50** of the thermocycling adaptor, shaped in a truncated pyramidal form. The tip **50** is used to penetrate the central opening **42, 43** of the cartridge **40** and apply forces to the sensor carrier **11, 21.** The tip **50** touches the window area **6** of the sensor carrier **11, 21** on its contact surface **51.** The tip **50** may also contain a thermoelectric element **61** for heating or cooling of the sensor carrier **11, 21** to control the temperature of the fluid within the chamber **32** of the sensor arrangement **207.**

**Fig. 8** shows a section view of sandwich **207,** formed by a first sensor carrier **11,** a gasket **31,** and a second sensor carrier **21,** enclosed in the cartridge **40** formed by two holder elements **41, 41',** and compressed in the window area **6** of the sandwich by two tips **50, 60.** Both tips **50, 60** are inserted in the central openings **42, 43** of the holder elements **41, 41'.** A chamber **32** is formed between the two films **1, 1'** and the inner edge **37** of the gasket **31.** The reaction fluid can be filled into the chamber **32** via the fluidic channels **44, 45** of the holder element, and via the fluidic openings **33, 34** in the films **1, 1'.** One of the tips **50, 60** contains a thermoelectric element **61.** Due to the material properties and the small thickness of the films, it is possible to thermally affect the analyte fluid **210** inside the chamber **32** by contacting the outer sides of the arrangement in the window area **6** with actively heated and/or cooled tips **50, 60.** The thermal energy will be conductively transferred through the film **1, 1'** wall to the fluid within the chamber **32** and vice versa, with the effect focused on the window area **6** of the films only, so rapid local heating and cooling can be achieved. This is preferable for the PCR amplification of the DNA.

In **Fig. 9a** is shown a section view of the cartridge **40** formed by two holder elements **41, 41'** and the sensor arrangement **207,** and the compression of the sensor arrangement **207** by two tips **50, 60.**

In **Fig. 9b** is shown a section view of the cartridge **40** with the fluidic channels **44, 45** in the holder elements **41.** Pockets **208** for sealing O-rings are made on both ends of the fluidic channels.

**Fig. 10a** shows an alternative arrangement of a sensor arrangement **247** with a film-gasket-film sandwich structure, where one of the sensor carriers **11** comprises spacers **71** on its sensor surface **204.** When the sensor arrangement **247** is compressed, the minimum distance between the sensor surfaces **204 of** the two films **1, 1'** is defined by the height of the spacers **71** over the base surface of the film **1.** The minimum distance between the sensor surfaces **204, 204'** of the films **1, 1'** of the first and second sensor carriers **11, 21** of the sensor arrangement **247** is limited by spacers **71** made on one of the films **1,** where the distance of the films **1, 1'** can not be less than the thickness of the spacers **71.** When the films **1, 1'** are compressed, the minimum distance between their sensor surfaces **201, 204'** are defined by the height of the spacers **71** over the sensor side surface of the film **1.**

In **Fig. 10b** is shown a further alternative embodiment of the film-gasket-film sandwich sensor arrangement **227,** where particles **70,** e.g. beads, are dispersed in the reaction fluid. When the sandwich sensor arrangement **227** is compressed together, the minimum distance between the sensor surfaces **204, 204'** of the films **1, 1'** of the first and second sensor carriers **11, 21** of the sensor arrangement **227** is limited by the respective diameters of the dispersed particles **70** in the reaction fluids.

**Fig.10c** illustrates another alternative embodiment of the film-gasket-film sandwich structured sensor arrangement **237,** where the second film **1"** has a substantially higher flexural rigidity than the first film **1.** Typically, the first film **1** of the arrangement **237** can be made of a 150 µm thick polyester film, and the second film **1"** is made of a more rigid and/or substantially thicker material such as a polycarbonate plate. In this example, the second film has a flexural modulus of 500 GPa, where films of even higher flexural rigidity may be used. When the sandwich style sensor arrangement **237** is compressed, only the film **1** of the first sensor carrier **11** with the lower flexural rigidity is deflected whereas the other film **1"** remains in its original state.

**Fig. 11** illustrates a section view of reader instrument **90** with a cartridge **40** placed in the mechanical adaptor with a clamping mechanism formed by an upper arm **91** and a lower arm **92.** Also shown is the thermocycling adaptor **93.**

**Fig.12** schematically illustrates an overview scheme of the entire reader instrument **90** with a cartridge **40** and the mechanical adaptor **95** or cartridge bed, the film distance adjustment adaptor **96,** the fluidic adaptor **94,** the measurement adaptor **97** with two electric multiplexers **217, 218,** and the instrument controller **98.**

To run an analyte detection assay the cartridge **40** with a film-gasket-film sandwich structured arrangement **207** is placed into the reader instrument **90.** Such reader instrument **90** comprises a housing in which the following principal components are arranged:

The reader instrument **90** comprises a mechanical adaptor **95** or cartridge bed which accommodates the cartridge **40** and enables mechanical compression of the film-gasket-film sandwich **207** between the two holder elements **41, 41',** thus sealing the volume of the chamber **32** in the arrangement **207.** The mechanical adaptor **95** is typically equipped with a manual, electromagnetic or other compression mechanism, such as a lever, electromagnet or similar.

The reader instrument **90** further comprises a fluidic adaptor **94** which enables delivery and removal of the analyte fluid **210** to and from the cartridge **40,** and thus to and from the chamber **32** in the sandwich arrangement **207.** The fluidic adapter **94** is equipped with suitable reagent reservoir **941,** fluid delivery mechanisms **942** such as pumps, connecting tubing and valves, and fluidic interfaces **943, 944** to the cartridge **40** and a second reservoir **945** for collecting the used fluidic reagents. The specific arrangement **207** of the fluidic adapter depends on the type of the assay.

The reader instrument **90** further comprises a film distance adjustment adaptor **96,** including two tips **50, 60** as described in **Fig. 7** and **Fig. 8** with planar bases which are inserted, from both sides, into the central openings **42, 43** of the holder elements **41, 41'** of the cartridge **40,** until they get into contact with the non-sensor surfaces **205, 205'** of the films **1, 1'.** The film distance adjustment adaptor **96** further includes a manual and/or mechatronic actuator, typically a piezoelectrically driven translation stage, which drives one of the tips **50, 60** and allows for positioning the tip **50, 60** in the direction normal to the sandwich plane, with positioning accuracy better than 1 µm. When one tip **50, 60** moves towards the film after getting into contact with its surface, it deflects one of the films **1, 1'.** The useful travel range of the tip **50, 60** is from the point of the first mechanical contact, up to the point where the sensor surface **204** of the first film **1** gets into mechanical contact with the sensor surface **204'** of the second film **1'** in the sandwich **207.** Since the deformation of the film **1** is elastic and therefore reversible, the film **1** returns to its unloaded, and thus planar, state after detracting the one tip **50, 60.**

Finally, the reader instrument **90** comprises a measurement adaptor **97.** If impedance and/or capacitance detection is used, the measurement adaptor **97** consists of two electrical multiplexers **217, 218,** the first multiplexer **217** selecting one of the electrical connections to the sensor elements **4** on the first sensor carrier **11** in the sandwich **207,** and the second multiplexer **218** selecting the electrical connections to the sensor elements **4** on the second sensor carrier **21** of the arrangement **207.** The combination of two selected sensor elements **4,** each of those being arranged on one of the sensor carriers **11, 21,** then represents a specific junction in the matrix of junctions. The measurement adaptor **97** further includes an impedance and/or capacitance measurement circuit **219,** which measures impedance and/or capacitance between the two selected sensor elements **4,** i.e. of the selected junction.

For alternative readout methods, an alternative embodiment of the reader instrument **90** can be equipped with alternative measurement adaptors, such as optical scanner or similar.

The reader instrument **90** has a thermocycling adaptor **93.** If the assay involves PCR amplification, the thermocycling adaptor **93** provides periodic heating and cooling with defined temperature-time steps. The thermocycling adaptor **93** typically includes a thermoelectric element **61,** for instance a Peltier element, which is attached with one side to an air cooler and with the other side to the tip **50.** The tip **50** is inserted into the central opening **42** of one holder elements **41** of the cartridge **40,** until it gets into contact with the non-sensor surface **205** of one of the sensor carriers **11, 21.** When the tip **50** is heated or cooled, the thermal energy is conductively transferred to the film **1** and thus to the analyte fluid **210** in the chamber **32** in the sandwich **207.** It is of advantage to build the tip **50** in a truncated conical or pyramidal form with one base being adapted to the form of the thermoelectric element **61,** and with the other base being adapted to the form of the window area **6** of the film **1.** This design allows for optimal conductive heat transfer and reduced energy loss. The tip **50** used for heating and cooling can, at the same time, be part of the distance adjustment adaptor **96** and fulfill both functions.

The reader instrument **90** further comprises an instrument controller **98** which is typically a microprocessor unit that controls the automated assay and readout process and communicates the measured data to downstream data processing, analysis and storage systems. The instrument controller is connected to the fluidic adaptor **94,** distance adjustment adaptor **96** and the measurement adaptor **97,** and can therefore control the processes of measurement.

If the analyte fluid **210** is filled in the chamber **32** through the fluidic openings **33, 34,** it gets into contact with the window area **6** of both sensor carriers **11, 21,** and thus to the sensor elements **4** and their functionalized surfaces **9,** so a simultaneous binding or hybridization reaction between analyte molecules and probe reaction partners can occur.

In a typical application, the probe reaction partners are oligonucleotides which can specifically hybridize with DNA strands present in the analyte. Since, typically, the DNA strands are substantially longer than the oligonucleotides, the DNA strands can hybridize with more than one oligonucleotide simultaneously. For example, a DNA strand in the analyte can hybridize with one oligonucleotide on its 3'-end (this oligonucleotide will be called 3'-end or forward probe in the following), which is with its 5'-end covalently bound at one surface, and with another oligonucleotide of opposite directionality on its 5'-end (in the following, this oligonucleotide will be called 5'-end or reverse probe) which is with its 3'-end covalently bound at the other surface. Only specific DNA strands (in particular, those with their nucleotide sequences matching the 3'-end probe and 5'-end probe molecules) hybridize as a function of temperature. If the 3'-end probe and the 5'-end probe are immobilized on the surfaces of the biosensor, which are exposed to the analyte, under suitable balanced physical and chemical conditions, the complementary matching DNA strands in the analyte will first hybridize and form a bridge between the 3'-end probes and the 5'-end probes. Thus, only these DNA strands will remain bound to the biosensor surface through the 3'-end and 5'-end probes. When the rest of the analyte is removed (typically, washed away from the surface), the remaining DNA molecules can be detected and/or quantified. If more than one 3'-end probe type and more than one 5'-end probe type are immobilized on the surface, different combinations of 3'-end and 5'-end hybridizations are possible (assuming respective matching DNA strands are present in the analyte). If the specific 3'-end probe and 5'-end probe molecules are immobilized on separate sites of the biosensor, then the matching DNA strands predominantly bind at these sites only. Since the site is specific for a given hybridization type, selective hybridization detection and/or quantification is possible.

The hybridization of DNA sequences can only take place if the distance of the immobilized 3'-end probe molecule to the 5'-end probe molecule is shorter than the length of the respectively matching DNA strand. Thus, additional selection of the matching DNA strands is possible, based on specific mutual distance between the immobilized 3'-end and 5'-end probe molecules.

It is possible to immobilize the probe reaction partners, typically the 3'-end and 5'-end probes, at different surfaces of the sensor. In particular, it is possible to immobilize the primers on two planar surfaces, respectively, which are parallel to each other, and both are exposed to the analyte. If the distance of the parallel surfaces is less than the length of the matching DNA strand, then hybridization reaction between the two surfaces is possible. By controlling the distance between the parallel surfaces the upper limit of the DNA length which is able to hybridize can be specified.

For example, in a right-handed B-DNA double helix, the stacked base pairs are separated by about 3.24 angstroms (0.324 nm). Therefore, the axial length of 10·000 base pairs long b-DNA double helix is 10·000×0.324 nm = 3·240 nm. If the distance between the two surfaces is about 3 µm, hybridization as described above can in principle only occur for molecules longer than 10·000 base pairs. Additional limitations may also apply.

For a multiplex study, it is of advantage to immobilize the different 3'-end and 5'-end probes in linear structures on the first and the second parallel surface, respectively. If the linear structures (stripes, lines, etc.) are arranged in a way that all rows on the first surface cross all columns on the second surface, then any junction (crossing) between any row on the first surface and any column on the second surface is a specific reaction site with specific 3'-end a 5'-end probes. Such junctions form a mesh (matrix), with rows represented by the lines of the first surface, and the columns represented by the lines of the second surface. Although, in theory, a reaction may take place between any probe immobilized along a given first-surface line, and any probe immobilized along a given second surface line, for sterical reasons only the junction with the minimum distance between the given lines is of practical importance. Thus, an assay with simultaneous hybridizations in different junctions within the said matrix is possible.

Several principles are known in the state-of-the-art to detect and/or quantify an outcome of the hybridization on one surface or between two surfaces. Labeled and unlabeled readout principles can be used for detection. In a labeled method, the single or double strand DNA is amplified by using chemical or physical labeled agent (reporter). The chemical or physical agents are then utilized for detection. Typically, luminescent molecules become chemically attached to unidentified DNA strands in the analyte solution. The assay is performed with the aim to detect in the analyte the presence of specific DNA strands, which only hybridize with specific 3'-end and 5'-end probes. The analyte is incubated in a chamber with two surfaces which contain attached probes as described above. The hybridization reaction is only enabled at the respectively matching probes, i.e. only in specific junctions of the matrix. After an incubation period, the rest of the analyte (containing DNA molecules which did not match any of the probes in the given junction matrix) is washed away. The matching DNA strands, which are already labeled with luminescent agents, remain bound to both, the first or the second surfaces at specific junctions. At suitable excitation, the luminescent labels emit observable radiation (typically, at visible light wavelengths), and the said junctions (i.e. matching probe pairs) can be identified.

It is further possible to improve the level of detection and/or enhance the quantification range by selective amplification. Typically, amplification of DNA strands can be achieved by polymerase chain reaction (PCR). In contrast to a 5'-end hybridization probe a 3'-end primer must be used which is covalently bound by its 5'-end and can be extended at its free 3'-site. 3'-primers like probes are always covalently bound by their 5'-ends.

Detection methods based on using various labels (reporter molecules) have been proven to be time-consuming, expensive and difficult to implement. However, similar detection and molecule identification is also possible without preliminary labeling the molecules in the analyte and/or other reaction components. The label-free detection and quantification methods use the intrinsic chemical or physical properties of the DNA strands themselves. Typically, DNA strands in the analyte are amplified prior to detection, as the change in chemical and/or physical properties of the DNA strands (or of the reaction environment) related to the presence of single (or a low number of) DNA molecules is too weak to be detected, so the label-free detection is typically connected with preceded or subsequent amplification.

A typical label-free detection method known in the state-of-the-art uses the local change in electrical impedance of the analyte liquid in the site where the DNA strands cumulate. This is due to substantial change in dielectric constant of a concentrated DNA (or similar organic polymer) solution as compared to a solution which does not contain DNA or similar compound. Therefore, measuring of the local electrical impedance can be used to identify the sites with concentrated DNA strands. Electrical impedance spectroscopy (EIS) is typically being used to detect the frequency-dependent impedance pattern of the junction.

More specifically, the impedance between two isolated electrodes which build one junction is of capacitance type. Therefore, the capacitance between two isolated electrodes, which are arranged in such way that the junction is filled with potentially amplified DNA strands can be measured. DNA strands can be a substantial part of the dielectric within the junction area. The capacitance measurement can be used to detect and/or quantify the DNA content. The electrodes are isolated, since the dielectric between them is typically formed by water or an electrolyte solution, which is required for hybridization and/or amplification of the DNA. Since the capacitance of a simple plate capacitor is directly proportional to the area of the plates and indirectly proportional to their distance, it is important to arrange the electrodes so that there are very close to each other, thus increasing the nominal capacitance to the range typical for standard capacitance measurement techniques in the electronics.

If the capacitance measurement technique is to be used for the readout, it is of advantage to combine the arrangement of the electrical conductors 2 to be used for capacitance measurement with the surfaces to be used for immobilizing the 3'-end probes/primers and the 5'-end probes/primers. The linear structures arranged on the first surface and embedding the 3'-end probes/primers, and linear structures arranged on the second surface and embedding the 5'-end probes/primers, can be made in such a way that they can be used as electrodes of the capacitors. More specifically, the linear structures can be made as linear, typically metallic, conductors, embedded partially or fully in the first and second surface, and covered by an insulating layer which separates the conducting metal from the volume occupied by the fluid analyte. The insulating layer can, simultaneously, serve as a layer containing chemical binding sites where the 3'-end probes/primers and the 5'-end probes/primers can be immobilized. Thus, the reaction partners are immobilized directly above the electrode. If the first and the second surfaces are approached to each other to a suitable distance, then conditions are created for the hybridization reaction to take place, and, simultaneously, a capacitor is created with a dielectric directly dependent from the amount of the reaction product.

Alternatively to the readout using electrical impedance/capacitance, a similar film can be used with other readout techniques. Optical readout can be used if the (unidentified) DNA strands in the analyte are labeled with luminescent agents as described above. The DNA strands matching any of the probe/primer combinations in the matrix junctions remain bound to both the first and the second surfaces at the said junctions. At suitable excitation, the luminescent labels emit observable radiation (typically, at visible light wavelengths). For this readout technique, a film is used with sensor elements not containing any metallic electrodes. Since the film is made from a transparent material, the luminescence originating in the junction on the sensor side of the film can be detected when observed from the non-sensor side of the film. Semiconductor lasers or multispectral lamps combined with band pass filters are used to excite the luminescence from the non-sensor side of the film, and standard luminescence detectors known in the art such as photomultipliers, CCD or CMOS cameras or similar are used to detect the luminescence on the non-sensor side of the film.

In the following, an example of an assay process is illustrated in detail with sensor carriers **11, 21,** an arrangement, a cartridge **40** and a reader instrument **90** as described above.

The first and the second functionalized sensor carriers **11, 21** and the gasket **31** are arranged in a sandwich structured arrangement **207,** where the sensor surfaces **204, 204'** of the films **1, 1'** of the sensor carriers **11, 21** are facing each other and are at the inner surface of the chamber **32** formed between the sensor carriers **11, 21** and the gasket **31.** The arrangement **207** is placed in a cartridge **40** comprising two holder elements **41, 41'.**

Alternatively, the process of assembly can begin with placing a first sensor carrier **11** on a lower holder element **41** containing positioning pins **209.** The film **1** of the first sensor carrier **11** is attached to the lower holder element **41,** with the sensor surface **204** facing up, being aligned with the positioning pins **209.** Then, the gasket **31,** the second sensor carrier **21** are attached, the sensor surface **204'** of the second sensor carrier **21** facing down towards the chamber **32.** The upper holder element **41'** is attached to the lower holder element **41** forming the cartridge **40.** The lower holder element **41** and upper holder element **41'** are held together by magnetic forces of the permanent magnets **214.**

The cartridge **40** is placed into the reader instrument **90** and is aligned in the mechanical adaptor **95** or cartridge bed. The connector pads **201** of the first film **1** and the second film **1'** of the sensor arrangement **207** are electrically connected with the measurement adaptor **97** of the reader instrument. Each of the sensor elements **4** of the first film **1** is connected to one of the inputs of the first multiplexer **217** of the measurement adaptor **97,** and each of the sensor elements **4** of the second film **1'** is connected to the inputs of the second multiplexer **218** of the measurement adaptor **97.** In this very example analogue multiplexers are used as first multiplexer **217** and second multiplexer **218.**

The cartridge **40** is fluidically connected to the fluidic adaptor **94** of the reader instrument **90.** This is typically accomplished simultaneously with sealing of the chamber **32** of the arrangement **207,** and with sealing of the fluidic connection between the sensor carriers **11, 21** of the arrangement **207** and film ends of the fluidic channels **44, 45** in the holder element **41,** and with sealing of the fluidic connection between the instrument ends of the fluidic channels **44, 45** in the holder element **41,** and the fluidic channels on the reader instrument **90.** The described sealing effects are simply achieved by exerting compression force to the holder elements **41, 41'** of the cartridge **40** in the direction normal to the plane of the films **1, 1'** of the sensor carriers **11, 21.** The compression is done by manually, electromagnetically, or other similarly driven clamping mechanism.

The analyte fluid **210** is filled into the chamber **32** of the sandwich **207** using the fluidic adaptor **94.** Since, in relaxed or unloaded state, the distance between the first film **1** and the second film **1'** is substantially higher than the distance necessary for chemical reaction between the two surfaces, it is substantially easier to fill the analyte fluid **210** into the chamber.

The film distance adjustment adaptor **96** is used to set up the distance between the sensor surfaces **204, 204'** of the films **1, 1'** of the first sensor carrier **11** and the second sensor carrier **21** of the sensor arrangement **207** to a value which is optimal for the hybridization or the other required reaction. The distance adjustment is enabled by the relatively low flexural modulus of the material of the film **1** of the sensor carriers **11, 21,** which can be deformed easily, e.g. like a thin membrane, while clamped at the margins between the gasket **31** and holder element **41.** The force required to deform and/or deflect the film **1, 1'** in the direction normal to the film surface is generated by the contact of the tip **50, 60** to the film **1,** and moving of the tip **50, 60** in the direction towards the second film **1'.** In this way, the films **1, 1'** of the sensor carriers **11, 21** approach each other, and the volume of the chamber **32** decreases, i.e. a negative deformation direction. Alternatively, the deflection can also be forced by pressure difference between the analyte fluid **210** on the sensor surface **204** of the film **1, 1',** and the air or any other medium on the non-sensor surface **205** of the film **1, 1'.** In this way, deflection in both positive and negative direction, i.e. increasing and decreasing the volume of the chamber **32,** respectively, is possible.

Several effects can be achieved by the elastic deflection of the films **1, 1':** If the films **1, 1'** get closer to each other, the diffusion path length for the molecules from the fluid phase of the volume in the chamber **32** to the direction normal to one of the sensor surface **204, 204'** gets shorter, which accelerates the reaction. To enable specific reaction type, such as a simultaneous binding of a molecule to the sensor compounds **5** immobilized at both surfaces **204, 204'** of the films **1, 1',** the distance should not exceed specific value so approaching of the films **1, 1'** is of principal importance. If impedance and/or capacitance measurement is used a readout method, the distance between the film **1, 1'** is affecting the baseline impedance and/or capacitance of the capacitor formed between the electrical conductors **2, 2'.** If this distance is increased, the electric fields between the conductors **2, 2'** might get shielded through formation of electric double layers formed by ions in solution to such extent that effective measurement of differences caused by local deposition of detected substances becomes unfeasible.

If PCR amplification is required, the thermocycling adaptor **93** is activated, and the analyte fluid **210,** which contains also all components required for PCR like polymerase, dNTP and further additives, in the chamber **32** of the sandwich **207** is periodically heated and cooled according to a specific time/temperature pattern, which leads to amplification of the specifically hybridized molecules.

Additional fluidic processes, such as exchanging the reaction fluid after partial PCR amplification, washing, etc., can be applied during the assay if specified. In particular, the reagents contained in the analyte which did not participate at the reaction and have not been bound by the immobilized primers, can be removed from the chamber **32.**

After PCR amplification, the amplicons are concentrated and bound in the junctions of the junction matrix with matching 5'-end and 3'-end primers. Different matching amplicons can be concentrated in a single junction, while some junctions may remain empty. The concentration of the amplicons in different junctions is, primarily, dependent on the original concentration of the potentially matching reaction partners in the analyte.

The measurement adaptor **97** is activated. The first multiplexer **217** connects the first sensor element **4** of the first film **1** to the first input of the impedance and/or capacitance measurement circuit **219.** The second multiplexer **218** connects the first sensor element **4** of the second film **1'** to the second input of the impedance and/or capacitance measurement circuit **219.** Impedance and/or capacitance is measured in this configuration, giving the data for the 'first sensor element-first sensor element' junction. This measurement readout is stored. The first multiplexer **217** then connects the second sensor element **4** of the first film **1** to the first input of the impedance and/or capacitance measurement circuit **219,** and the impedance and/or capacitance is measured and stored for the 'second sensor element-first sensor element' junction. The process is repeated for all sensor elements **4** of the first film **1** and all sensor elements **4** of the second film **1',** so impedance and/or capacitance readouts are stored for all junctions of the junction matrix.

The readouts are analyzed with respect to the known allocation of 3'-end and 5'-end probes/primers on the sensor elements **4** of the first film **1** and the second film **1',** and detection and/or quantification results are produced.

In the following, an alternative **second example** of the invention is provided by template specific PCR amplification in an assembly of two ultra high density flexible circuit boards with functionalized electrical conductors **2, 2'.** Unless otherwise stated, this alternative example is identical with the first example.

Structured PET films which contain 24 electrical conductors **2** each were designed as described in the first example of the invention. The film **1** was heat treated polyethylene terephthalate (PET) substrate. The electrical conductors **2** were made of nickel carrying a top layer of gold, and measured 25 µm in width and approximately 11 mm in length inside the window area **6.** The electrical conductors **2** are leading from the window area **6** to the connector pads **201** of the electrical connector **3.** The surfaces of the PET support and electrical conductors **2, 2'** formed by metal of the electrodes were on level which means that the surface of the structured PET films is entirely flat.

The electrical conductors **2, 2'** are electrically insulated and functionalized with different sensor compounds **5,** namely oligonucleotides functioning as PCR primers, through a combination of electrode guided electrodeposition and polymerization. The sensor compounds **5,** in this example primer molecules are used, are bound to the polymer via spacer molecules and therefore surface exposed and accessible.

For the coating process a cartridge **40** of 50x50x20.5 mm outer dimension analogue to the description in the first example has been assembled containing one 24 electrode PET film **1, 1',** one 250 µm thick gasket **31** and one stainless steel counter electrode. A zero-insertion force electrical connector **3** and flat cable was used to electrically connect the film **1, 1'** to a switch board through which one or several electrodes could be set to a predetermined potential. The chamber **32** in the window area **6** confined a volume of approximately 25 µl. The solutions for the functionalization were successively channeled through the chamber **32.** During an exposure time the functionalization of specific electrical conductors **2, 2'** was carried out. Between each functionalization the chamber **32** has been rinsed thoroughly with water. After the functionalization the cartridge has been disassembled to remove the functionalized film **1, 1'** before rinsing it thoroughly with water, drying it with a stream of nitrogen and placing it into an oven for curing. The coating was inspected by fluorescence scanning and exemplarily by AFM scanning. Electrodes were homogenously coated with functionalizing films of approximately 1 µm thickness.

Then, a sensor arrangement **207** comprising two of such individually functionalized sensor carriers **1, 1'** was assembled with a 250 µm thick gasket in a cartridge **40** as described in the first example. The cartridge **40** was transferred into, and aligned by the mechanical adaptor of the reader instrument **90.** The connector pads **201** of the electrical connectors **3** were electrically connected with the measurement adaptor **97** of the reader instrument **90.**

The fluidic channels **44, 45** connect the outer side of the cartridge **40** with the chamber in the arrangement **207.** Only the fluidic channels **45** of the upper holder **41'** are used, the connection between the film and the lower holder **41** is blinded by replacing a sealing O-ring with a matching size silicone disc. Then, the outer side of the cartridge **40** was connected to the fluidic adaptor **94** of the reader instrument. Through controlled pumping of solutions either a continuous stream of liquid or aliquots in the order of 25 µl at the time could be flushed through or injected into the chamber **32.** The aqueous solutions contained buffer compounds, template DNA or a PCR mix with TaqHS polymerase, dNTP and additives.

The electrical connectors **3** of the first and the second film **1, 1'** of the sensor arrangement **207** are connected via zero-insertion force connectors. A relaxed state, where the two flexible functionalized films had no physical contact, and a loaded state, where the two flexible functionalized films were in physical contact, could be imposed alternatively through a piezo-driven height manipulation device (distance adjustment adaptor **96**) which was brought and stayed in contact with the upper film. The lower film was in contact with a Peltier element connected metal block, the thermocycling adaptor **93.**

The relaxed state was used to facilitate fast solution exchange during washing and filling of the measurement cell. The separation between the two films **1, 1'** in the centre and around the edges was approximately 250 µm similar to the height of the gasket. Under this condition solutions could pass the cell with flow rates of approximately 1 ml/min which accounts for approximately one nominal exchange of the volume in 1.5 sec. Tests with colored solutions have shown that solution exchanges proceed rather evenly across the entire surface in the open state, slightly faster along the central axis between the inlet and outlet port, slightly slower along the edges, slowest at the corners. In the loaded state solution exchange would proceed significantly slower and essentially only along the edges where the first and seconds films **1, 1'** diverge to the distance which is determined by the height of the gasket, here 250 µm. The difference of the solution exchange rate between the middle and the edge of the window area **6** would be very large. Therefore, in the unloaded state the sensor arrangement **207** was first washed with ultrapure water before it was filled with 25 µl of a solution containing 1.5 ng/µl DNA template sequence of 350 bp length, 0.08 u TaqHS polymerase, and 0.1 mM dNTP, 0.2 µg/ml BSA, 3 mM Mg²⁺, 0.3 M Trehalose in a Taq Hotstar buffer (Cryogene, US). The template contained complementary sequences to the primers F and R. The primers N were not complementary to partial sequences within the template.

Then, the loaded state was applied through the piezo driven positioning system, the film distance adjustment adaptor **96,** which moved the first film **1** to reach close contact with the second film **1'.** Since the electrical conductors **2, 2'** are embedded in the supporting PET polymer, and are surmounted just by the functionalizing coating layer, the approach of the surfaces stopped when the curved coatings touch each other. Each electrical conductor **2** of the first film contacts each electrical conductor **2'** of the second film in a very small point shaped area, thereby forming a single sensor. Small deviations from the perfect even flat surface alignment are mediated by a thin gel layer which is situated between the first film **1** and the tip **50.** The gel layer levels small height variations and ensures that each electrode junction is compressed with a moderate pressure which is small enough to not indent the functionalized coating completely.

The reference impedances of all combinations of electrical conductors **2, 2'** from the first and second film **1, 1'** were measured successively with a scanning routine which set all non-participating electrical conductors **2, 2'** on shielding ground.

The outcome of the measurement is typically presented in form of an impedance matrix diagram. The rows R0 to R23 of the impedance matrix represent the electrical conductors **2** of the first film **1,** whereas the columns C0 to C23 of the impedance matrix represent the electrical conductors **2'** of the second film **1'.** The matrix cells stand for the individual junctions. Impedance is measured for all individual sensors of the sensor arrangement at a given frequency. In this example, a frequency of 1 MHz was used. The modulus component of each of the complex impedance values is determined.

In **Figs. 13a** and **13b****,** such diagrams are shown in low impedance resolution, discriminating three impedance modulus ranges only. In this example, threshold values of 5 MOhm, i.e. mega Ohms, and 10 MOhm are used. Sensors with an impedance value higher than 10 MOhm are depicted by white circles, sensors with an impedance modulus between 5 MOhm and 10 MOhm are depicted by concentrical circles and sensors with an impedance modulus lower than 5 MOhm are depicted by black circles. Higher impedance, i.e. lower capacitance at given measurement frequency, means higher concentration of the hybridized and amplified substances in the given sensor junction. Note that the impedance is actually measured with a substantially higher resolution, and the diagrams have been simplified for publication purposes.

The regular pattern of the impedance which is seen in **Fig. 13a** is caused by the different functionalization of the electrodes. The significant differences are seen between sensors elements **4** at junctions of electrodes which carry the primers, F, R or N, and junctions of electrical conductors **2, 2'** which have no primers in their respective coatings **9.** Further, also in the loaded state 40 PCR cycles were conducted by thermocycling the sensor arrangement **207** alternating between a denaturing step for 45 sec at 95°C, an annealing step for 30 sec at 57°C and an extension step for 60 sec at 72°C. After the PCR cycling the second impedance scan was recorded comprising again all combinations of electrical conductors **2, 2'.** Results are shown in **Fig. 13b****.** The impedimetric scans before and after the PCR display a change in signal intensity as function of the ability of the primers to hybridize and amplify the particular template.

As explained above, in a typical application, the targeted molecules in the analyte hybridize to the forward primers F which are immobilized in the first film 1 with their respective 5'-site. During the first extension the primers F are extended in 5'- to 3'-direction and produce complementary copies of the analyte molecules which are now covalently bound to the first film **1** via the primer binding site. After the subsequent melting step and during the following annealing step the complementary copies are capable to hybridize to the reverse primers R which are immobilized in the second film **1'** with the opposite end. For a given molecule species, a junction containing matching forward primers in the first film **1** and matching reverse primers in the second film **1',** is denoted as "F-R", wherein the letter(s) before and after dash specifying substances in the first and second film **1, 1',** respectively. For experimental purposes, it is of advantage to check the general ability of the matching molecules to hybridize to the primers specifically and selectively, i.e., to hybridize to the matching primers irrespectively of their physical location, and not to bind to non-matching primers or to junctions with no primers. Therefore, in our experiments, we also used junctions which are functionalized as follows:
- 'FR-FR', i.e. both the first film and the second film contain a mixture of forward and reverse primers,
- 'FR-F' and 'FR-R' wherein the first film is functionalized with a primer mixture comprising forward and reverse primers, the second film only contains forward or reverse primer, respectively,
- 'FR-N', wherein the first film is functionalized with a forward and reverse primer mixture, the second film only contains a non-matching primer,
- 'F-F' ('R-R') wherein the first film is functionalized with a forward (reverse) primer only, and the second film is functionalized with a forward (reverse) primer only,
- 'F-N', forward primer in the first film, non-matching primer in the second film,
- 'R-N', reverse primer in the first film, non-matching primer in the second film,
- 'N-N', non matching primers in both films and
- '0-0' zero indicating that no primers are deposited on the respective coatings,
- as well as all combinations of any of the primers with no primers.

The combinatorial approach of the matrix structure implies that all possible primer combinations can be realized in symmetrical matrices. The statistical evaluation of all equivalent junctions from the pool of in total 576 junctions is shown as mean values with the respected confidence interval in **Fig. 14****.** The impedance modulus represents the change of the impedance in relation to the change of the dielectric inside the electrode junctions as a function of the present sensor or primer molecules. The largest changes are seen at junctions with combinations of F- and R-primers. The smallest change is seen at junctions without primers. Junctions which contain primers N which are not complement to the sequence of the template display also very little change. The results demonstrate the label free detection of DNA by impedimetric scanning of "nanogap junctions".

The highest concentration of the hybridized molecules at a given junction is expected for the 'FR-FR' configuration, since multiple hybridization combinations (first film-first film, second film-second film, first film-second film) are possible. **Fig.14** shows that this assumption is fulfilled. It is also shown that the first film-second film hybridization, which is the desired functionality is preferred over single surface hybridization, the 'F-R' junctions having higher mean impedance than the 'FR-F' junctions. The 'FR-R' junctions have higher mean impedance than the 'F-R', however a substantially higher deviation. The mean impedance modulus of any of the non-matching combinations, i.e. 'F-N', 'R-N', 'N-N', and '0-0', is significantly below the value for any of the matching configurations.

The disclosed examples of biosensors may be used for multiplex assays in molecular biology, in particular, for assays requiring selective detection and/or quantification of analyte constituents which react specifically with two probes simultaneously. The probes are part of the biosensor and are specific for a given biosensor type. If more than two probes are included in the biosensor, then the biosensor can be used for multiplexed studies, in that more than one combination of the probes can react with different analyte constituents, so more than one analyte constituent can be specifically detected and/or quantified simultaneously.

### References cited

### Journal Publications

Adessi C, Matton G, Ayala G, Turcatti G, Mermod JJ, Mayer P, Kawashima E,. 2000 Solid phase DNA amplification: characterisation of primer attachment and amplification mechanisms. Nucleic Acids Res. 28e87
Focke M, Kosse D, Müller C, Reinecke H, Zengerle R, von Stetten F., 2010 Lab-on-a-Foil: microfluidics on thin and flexible films. Lab Chip. 10(11):1365-86
Jia G, Siegrist J, Deng C, Zoval JV, Stewart G, Peytavi R, Huletsky A, Bergeron MG, Madou MJ. 2007 A low-cost, disposable card for rapid polymerase chain reaction. Colloids Surf B Biointerfaces. 58(1):52-60
Khan Z, Poetter K, Park DJ., 2008 Enhanced solid phase PCR: mechanisms to increase priming by solid support primers. AnalBiochem. 375(2):391-3
Liu Y, Rauch CB, Stevens RL, Lenigk R, Yang J, Rhine DB, Grodzinski P,. 2002 DNA amplification and hybridization assays in integrated plastic monolithic devices. Anal Chem. 74(13):3063-70
Saiki R, Scharf S, Faloona F, Mullis K, Horn G, Erlich H, Arnheim N,. 1985 Enzymatic amplification of beta-globin genomic sequences and restriction site analysis for diagnosis of sickle cell anemia. Science 230 (4732): 1350-1354

### Patent Publications

WO 2010104479, Electrical sensor for ultrasensitive nucleic acid detection
PCT/AT2012/000043, Junction array sensor device
US 6878345, Ultra high throughput bioassay screening system
US5863502A1, Parallel reaction cassette and associated devices
EP 1 415 710, Composite flexible array substrate

## Claims

1. Sensor arrangement (207, 227, 237, 247) comprising
- two sensor carriers (11, 21) each sensor carrier (11) comprising a film (1, 1') comprising a plurality of functionalized sensor elements (4) each comprising an functional layer (9) formed by a coating through which sensor compounds (5) are immobilized, wherein the functional layers (9) are located on the same surface (204, 204') of the film (1, 1') within a window area (6), wherein a region (7, 7') of each of the functional layers (9) of the sensor elements (4) is functionalized with a one or more sensor compounds (5, 5'), the sensor compounds (5, 5') containing oligonucleotides binding to binding sites of analyte molecules, each functional layer having an individual functionalization coating and
- a gasket (31) comprising a central opening (38),
- wherein the film (1) of the first sensor carrier (11) is flexible,
wherein the gasket (31) is arranged between the two sensor carriers (11, 21), so that a chamber (32) is formed between the two sensor carriers (11, 21) and surrounded by the inner edge (37) of the gasket (31),
wherein the respective surfaces of the sensor carriers (11, 21), on which the sensor elements (4) are arranged, are facing each other and each sensor element (4) and/or conductor (2) of one of the sensor carriers (11, 21) opposes or faces at least one or each sensor element and/or conductor (2) of the opposite sensor carrier (21, 11), and
wherein the sensor arrangement comprises a plurality of electrical conductors (2), preferably comprising at least one line shaped portion, wherein each of the conductors (2) is assigned to one sensor element (4), and each conductor (2), preferably the line shaped portion thereof, is located underneath the functional layer (9) of the respective sensor element (4) in or on the film and/or second film (1, 1').

2. Sensor arrangement (207, 227, 237, 247) according claim 1, wherein material of the second film (1') has a flexural modulus higher than 5 Gpa.

3. A sensor arrangement according to claim 1 or 2, wherein the film and/or second film material has a flexural modulus from 0.5 GPa to 2 GPa, preferably from 0.75 GPa to 1.25 GPa, and/or
wherein the thickness of the film (1, 1') and/or second film is between 10 µm to 300 µm, in particular between 100µm to 200 µm, and/or
wherein the film (1) and/or second film is composed of a polymer, such as polyethylene, polyethylene terephtalate (PET), polyimide, polyethylene naphthalate, polycarbonate, polyether sulphone, polyarylate and/or mixtures thereof.

4. A sensor arrangement according to any of the preceding claims, wherein
the flexibility and elasticity of the material of the film (1) and/or second film, and/or
the flexibility and elasticity of the material of the functional layers (9), and/or
the size and/or shape of the window area (6),
are chosen so that, when the boundary of the window area (6) is fixed in direction normal to the plane of the film and/or second film (1, 1'), and the window area (6) is deflected and/or deformed, out of the plane of the film and/or second film (1, 1') in the direction normal to the plane of the film and/or second film (1, 1'), wherein at least one part of the surface is pushed by 10 to 500 µm, preferably by 20 to 200 µm, into the direction normal to the plane of the film and/or second film (1, 1'), the film and/or second film (1, 1') is deflected elastically, in particular without being damaged.

5. A sensor arrangement according to any of the preceding claims, wherein at least the window area (6) of the film is transparent and/or
that the window area (6) is arranged in the centre of the film and/or second film (1, 1').

6. A sensor arrangement according to any of the preceding claims **characterized in that** one or more spacers (71), preferably each having the form of a protrusion, are arranged on the surface of the sensor carrier (11, 21) on the side of the functional layers (9), wherein the spacers (71) are elevated of the surface of the sensor carrier (11, 21) by 0.05 µm to 1 µm, preferably by 0.2 µm to 0.5 µm.

7. A sensor arrangement according to any of the preceding claims, **characterized in that** the sensor compounds (5) contain organic polymers or DNA or RNA molecules.

8. A sensor arrangement according to any of the preceding claims,
wherein the sensor carrier preferably comprises an electrical connector (3), which is arranged on the film and/or second film (1, 1'), the electrical connector (3) comprising a number of connector pads, each of the connector pads being connected to one of the conductors (2), and/or
wherein the conductors (2) in or on the film and/or second film (1, 1') contain or consist of nickel, copper, gold, carbon, indium tin oxide, semiconductors or any other similar conducting material.

9. A sensor arrangement according to any of the preceding claims,
wherein the film and/or second film (1, 1') has a rectangular shape,
wherein the conductors (2) are arranged in parallel in at least the window area (6) of the film and/or second film (1, 1'), and
wherein at least one portion of each sensor element (4) and/or each conductor (2), preferably the respective functionalized region (7) of the sensor element (4), is inclined to the edges of the film and/or second film (1, 1'), preferably by an angle of 40° to 50°, especially 45°.

10. A sensor arrangement according to any of the preceding claims, wherein the second film (1') has a plurality of line shaped electrical conductors (2) and one electrical connector (3) and/or spacers (71) arranged on its surface.

11. Sensor arrangement (207, 227, 237, 247) according to any of the preceding claims,
wherein particles (70) or beads are arranged between the sensitive opposing surfaces (204, 204') of the sensor carriers within the chamber (32),
wherein the particles (70) or beads are preferably included or dispersed in the analyte fluid (210) containing analyte molecules to by analyzed,
wherein the minimum distance between the sensor surfaces (204, 204') of the two films (1, 1') is defined by the diameter of the particles (70) or beads.

12. Sensor arrangement (207, 227, 237, 247) according to any of the preceding claims, **characterized by** an inlet (33) and an outlet (34) leading through one or both of the sensor carriers (11, 21) and/or in the gasket (31), leading from the outside to the chamber (32) and enabling fluid to stream into the chamber (32) or out of the chamber (32) through the inlet (33) and the outlet (34).

13. Cartridge (40) comprising
- an arrangement (207, 227, 237, 247) according to according to any of the preceding claims and
- at least one, preferably two, holder elements (41),
wherein the gasket (31) and the sensor carriers (11, 21) or one sensor carrier (11) and one film (1') are fixed and compressed by the holder elements (41) so that the chamber (32) is confined by the gasket (31), and/or the sensor carriers (11, 21) and/or the film (1, 1'), so that the chamber (32) is sealed and impermeable to liquid.

14. Cartridge (40) according to claim 13, wherein the holder element (41) has at least one opening (42), wherein the opening (42) is covered by one part of one of the sensor carriers (11, 21), preferably by the window area (6), said part at least partially confining the chamber (32).

15. Cartridge (40) according to claim 13 or 14,
wherein the holder element (41) has two opposing openings (42, 43), wherein the first opening (42) is covered by one portion of one of the sensor carriers (11, 21), which is preferably the window area (6) of said sensor carrier (11, 21), and
wherein the second opening (43) is covered by one portion of the other sensor carrier (11, 12) or film,
wherein the second opening (43) is preferably covered by the window area (6) of the other sensor carrier (11, 21) or film and/or second film (1, 1'), said portions of the sensor carriers (11, 21) or film at least partially confining the chamber (32).

16. Cartridge (40) according to one of the claims 13 to 15,
wherein the holder element (41) further comprises at least two channels (44, 45),
wherein the first channel (44) connects the inlet (33) with the outer surface of the holder element (41) and the second channel (45) connects the outlet (34) with the outer surface of the holder element (41).

17. Cartridge (40) according to one of the claims 13 to 16 comprising two holder elements (41), wherein
a) the sensor carriers (11, 21) and the gasket (31), or
b) the sensor carrier (11) and one film (1') and the gasket (31),
are fixed between the holder elements (41),
wherein the holder elements (41) preferably comprise protrusions (209), particularly positioning pins, and holes (47), matching each other when the holder elements (41) are assembled, and
wherein the sensor carriers (11, 21), the film and/or the gasket (31) have positioning holes (12, 22, 39) being penetrated by at least some of the protrusions (209).

18. Cartridge (40) according to one of the claims 13 to 17 comprising two holder elements (41), the holder elements (41) compressing the arrangement (207, 227, 230, 247) to seal the chamber (32).

19. Method for the measurement of a concentration of the analyte compound in an analyte fluid (210) using nucleic acid amplification, with a sensor arrangement (207, 227, 237, 247) or a cartridge (40) according to one of the preceding claims,
a) wherein an elastically flexible first film (1) and second film (1') are provided, a first sensor compound (5) being arranged on a region (7) of the first film (1) and a second sensor compound (5') being arranged on a region (7') of the second film (1'), the sensor compounds (5, 5') being arranged on opposing sensor surfaces (204, 204') of the films (1,1') facing each other,
b) wherein the first sensor compound (5) binds or adheres to a first portion of the molecule of the analyte compound and the second sensor compound (5') binds or adheres to a second portion of the molecule of the analyte compound, and
c) wherein the analyte fluid (210) is arranged in a chamber (32) formed between and surrounded by the two films (1, 1'),
d) wherein the first film (1) and the second film (1') are elastically deflected or deformed in a manner, that the distance between the opposing surfaces of the films (1, 1') facing each other is reduced and equals or is smaller than the distance between the first portion and the second portion of the molecule of the analyte compound,
e) so that a sensor is formed between the region (7) of the first film (1) on which the first sensor compound (5) is arranged and the region (7') of the second film (1) on which the second sensor compound (5') is arranged, where said regions (7, 7') are arranged to face each other, and
f) wherein the amount of molecules bound or adhered to the first and second sensor compounds (5, 5') is measured, wherein the capacitance or impedance between the electrical conductors (2) in the regions (7, 7') of the first film (1) and the second film (1') on which sensor compounds (5, 5') are arranged is measured, and said capacitance or impedance indicating the amount of the analyte compound in the analyte fluid (210).

20. Method according to claim 19, wherein particles (70) or beads of a given diameter are added to the analyte fluid (210), wherein the particles (70) or beads are filled into the chamber (32) between the two films (1, 1'), so that the minimum distance between the sensor surfaces (204, 204') of the two films (1, 1') is defined by the diameter of the particles.

21. Method according to any of claims 19 to 20, with two sensor carriers (11, 21) according to one of the claims 1 to 8 each of which comprises one film (1, 1') or with an arrangement according to one of the claims 9 to 13 or a cartridge (40) according to one of the claims 14 to 19 comprising the films (1, 1'),
wherein a plurality of sensors is formed between the regions (7) of the first film (1) on which the first sensor compound (5) or plurality thereof is arranged and the regions (7') of the second film (1') on which the second sensor compound (5') or plurality thereof is arranged, where said regions (7, 7') are arranged to face each other, and
wherein the concentration of analyte compounds located or deposited between two facing regions (7, 7') of a sensor is determined separately for each sensor.

22. Method according to any of claims 19 to 21, wherein before setting the distance of the opposing films (1, 1') the distance of the films (1, 1') is increased in order to increase the amount of fluid within the chamber (32) and/or
the distance of the films (1, 1') is regulated to adjust the internal volume of the chamber (32) in order to control the amount of analyte fluid (210) accommodated in the chamber (32).

23. Method according to any of claims 19 to 22,
wherein the distance between the films (1, 1') is, preferably repeatedly, changed before or during the measurement so that the analyte fluid (210) in the chamber (32) is mechanically agitated, so that by adjusting the flow in chamber (32) a specific shear force distribution profile is achieved in the chamber (32).

24. Method according to any of claims 19 to 23, wherein the distance between the films (1, 1') is changed in order to pump fluid into the chamber (32) or out of the chamber (32).

## Patentansprüche

1. Sensoranordnung (207, 227, 237, 247) umfassend
- zwei Sensorträger (11, 21), wobei jeder Sensorträger (11) einen Film (1, 1') umfasst, der eine Vielzahl von funktionalisierten Sensorelementen (4) umfasst, die jeweils eine funktionelle Schicht (9) umfassen, die durch eine Beschichtung gebildet ist, durch welche die Sensorverbindungen (5) immobilisiert werden, wobei sich die funktionellen Schichten (9) auf derselben Oberfläche (204, 204') des Films (1, 1') innerhalb eines Fensterbereichs (6) befinden, wobei eine Region (7, 7') jeder der funktionellen Schichten (9) der Sensorelemente (4) mit einer oder mehreren Sensorverbindungen (5, 5') funktionalisiert ist, wobei die Sensorverbindungen (5, 5') Oligonukleotide enthalten, die an Bindungsstellen von Analytmolekülen binden, wobei jede funktionelle Schicht eine individuelle Funktionalisierungsbeschichtung aufweist und
- eine Dichtung (31), die eine zentrale Öffnung (38) umfasst,
- wobei der Film (1) des ersten Sensorträgers (11) flexibel ist,
wobei die Dichtung (31) zwischen den beiden Sensorträgern (11, 21) angeordnet ist, sodass eine Kammer (32) zwischen den beiden Sensorträgern (11, 21) gebildet wird und vom Innenrand (37) der Dichtung (31) umgeben ist,
wobei die entsprechenden Oberflächen der Sensorträger (11, 21), auf denen die Sensorelemente (4) angeordnet sind, einander zugewandt sind und jedes Sensorelement (4) und/oder jeder Leiter (2) von einem der Sensorträger (11, 21) zumindest einem oder jedem Sensorelement und/oder Leiter (2) des gegenüberliegenden Sensorträgers (21, 11) gegenüberliegt oder zugewandt ist, und
wobei die Sensoranordnung eine Vielzahl von elektrischen Leitern (2) umfasst, die vorzugsweise zumindest einen linienförmigen Abschnitt umfassen, wobei jeder der Leiter (2) einem Sensorelement (4) zugeordnet ist und sich jeder Leiter (2), vorzugsweise sein linienförmiger Abschnitt, unter der funktionellen Schicht (9) des entsprechenden Sensorelements (4) in oder auf dem Film und/oder zweiten Film (1, 1') befindet.

2. Sensoranordnung (207, 227, 237, 247) nach Anspruch 1, wobei das Material des zweiten Films (1') einen Biegemodul über 5 GPa aufweist.

3. Sensoranordnung nach Anspruch 1 oder 2, wobei das Material des Films und/oder des zweiten Films einen Biegemodul von 0,5 GPa bis 2 GPa, vorzugsweise von 0,75 GPa bis 1,25 GPa, aufweist und/oder
wobei die Dicke des Films und/oder des zweiten Films (1, 1') zwischen 10 µm bis 300 µm, insbesondere zwischen 100 µm bis 200 µm, beträgt und/oder
wobei der Film (1) und/oder der zweite Film aus einem Polymer wie beispielsweise Polyethylen, Polyethylenterephtalat (PET), Polyimid, Polyethylennaphthalat, Polycarbonat, Polyethersulfon, Polyarylat und/oder Gemischen davon besteht.

4. Sensoranordnung nach einem der vorangehenden Ansprüche, worin
die Flexibilität und Elastizität des Materials des Films (1) und/oder des zweiten Films und/oder
die Flexibilität und Elastizität des Materials der funktionellen Schichten (9) und/oder die Größe und/oder Form des Fensterbereichs (6),
so gewählt sind, dass, wenn die Grenze des Fensterbereichs (6) in der Richtung normal zur Ebene des Films und/oder des zweiten Films (1, 1') fixiert ist und der Fensterbereich (6) aus der Ebene des Films und/oder zweiten Films (1, 1') heraus in die Richtung normal zur Ebene des Films und/oder zweiten Films (1, 1') gebogen und/oder verformt wird, wobei zumindest ein Teil der Oberfläche 10 bis 500 µm, vorzugsweise 20 bis 200 µm weit in die Richtung normal zur Ebene des Films und/oder zweiten Films (1, 1') gedrückt wird, der Film und/oder zweite Film (1, 1') elastisch verbogen wird, insbesondere ohne beschädigt zu werden.

5. Sensoranordnung nach einem der vorangehenden Ansprüche, wobei zumindest der Fensterbereich (6) des Films transparent ist und/oder
der Fensterbereich (6) im Zentrum des Films und/oder zweiten Films (1, 1') angeordnet ist.

6. Sensoranordnung nach einem der vorangehenden Ansprüche, **dadurch gekennzeichnet, dass** ein oder mehrere Abstandshalter (71), die vorzugsweise alle die Form eines Vorsprungs aufweisen, auf der Oberfläche des Sensorträgers (11, 21) auf der Seite der funktionellen Schichten (9) angeordnet sind, wobei die Abstandshalter (71) von der Oberfläche des Sensorträgers (11, 21) um 0,05 µm bis 1 µm, vorzugsweise um 0,2 µm bis 0,5 µm, erhöht sind.

7. Sensoranordnung nach einem der vorangehenden Ansprüche, **dadurch gekennzeichnet, dass** die Sensorverbindungen (5) organische Polymere oder DNA- oder RNA-Moleküle enthalten.

8. Sensoranordnung nach einem der vorangehenden Ansprüche,
wobei der Sensorträger vorzugsweise einen elektrischen Anschluss (3) umfasst, der auf dem Film und/oder zweiten Film (1, 1') angeordnet ist, wobei der elektrische Anschluss (3) eine Anzahl von Anschlussflächen umfasst, wobei jede der Anschlussflächen mit einem der Leiter (2) verbunden ist, und/oder
wobei die Leiter (2) in oder auf dem Film und/oder zweite Film (1, 1') Nickel, Kupfer, Gold, Kohlenstoff, Indiumzinnoxid, Halbleiter oder ein beliebiges anderes ähnliches leitendes Material enthalten oder daraus bestehen.

9. Sensoranordnung nach einem der vorangehenden Ansprüche,
wobei der Film und/oder zweite Film (1, 1') eine rechteckige Form aufweist,
wobei die Leiter (2) zumindest im Fensterbereich (6) des Films und/oder zweiten Films (1, 1') parallel angeordnet sind und
wobei zumindest ein Abschnitt jedes Sensorelements (4) und/oder jedes Leiters (2), vorzugsweise die jeweilige funktionalisierte Region (7) des Sensorelements (4), zu den Rändern des Films und/oder zweiten Films (1, 1') hin geneigt ist, vorzugsweise in einem Winkel von 40° bis 50°, insbesondere 45°.

10. Sensoranordnung nach einem der vorangehenden Ansprüche, wobei der zweite Film (1') eine Vielzahl von linienförmigen elektrischen Leitern (2) und einen elektrischen Leiter (3) und/oder Abstandshalter (71), die auf seiner Oberfläche angeordnet sind, aufweist.

11. Sensoranordnung (207, 227, 237, 247) nach einem der vorangehenden Ansprüche,
wobei Partikel (70) oder Kügelchen zwischen den empfindlichen gegenüberliegenden Oberflächen (204, 204') der Sensorträger innerhalb der Kammer (32) angeordnet sind, wobei die Partikel (70) oder Kügelchen vorzugsweise im Analytfluid (210) enthalten oder dispergiert sind, das zu analysierende Analytmoleküle enthält,
wobei der Mindestabstand zwischen den Sensoroberflächen (204, 204') der beiden Filme (1, 1') durch den Durchmesser der Partikel (70) oder Kügelchen definiert ist.

12. Sensoranordnung (207, 227, 237, 247) nach einem der vorangehenden Ansprüche, **gekennzeichnet durch** einen Einlass (33) und einen Auslass (34), die durch einen oder beide der Sensorträger (11, 21) und/oder in die Dichtung (31) führen, wobei sie von außen in die Kammer (32) führen und es Fluid ermöglichen, durch den Einlass (33) und den Auslass (34) in die Kammer (32) hinein oder aus der Kammer (32) heraus zu strömen.

13. Kassette (40), umfassend
- eine Anordnung (207, 227, 237, 247) nach einem der vorangehenden Ansprüche und
- zumindest ein, vorzugsweise zwei Halterelemente (41),
wobei die Dichtung (31) und die Sensorträger (11, 21) oder ein Sensorträger (11) und ein Film (1') durch die Halterelemente (41) fixiert und komprimiert sind, sodass die Kammer (32) durch die Dichtung (31) und/oder die Sensorträger (11, 21) und/oder den Film (1, 1') begrenzt ist, sodass die Kammer (32) versiegelt und flüssigkeitsundurchlässig ist.

14. Kassette (40) nach Anspruch 13, wobei das Halterelement (41) zumindest eine Öffnung (42) aufweist, wobei die Öffnung (42) durch einen Teil von einem der Sensorträger (11, 21) bedeckt ist, vorzugsweise vom Fensterbereich (6), wobei der Teil zumindest teilweise die Kammer (32) begrenzt.

15. Kassette (40) nach Anspruch 13 oder 14,
wobei das Halterelement (41) zwei gegenüberliegende Öffnungen (42, 43) aufweist, wobei die erste Öffnung (42) durch einen Teil von einem der Sensorträger (11, 21) bedeckt ist, der vorzugsweise der Fensterbereich (6) des Sensorträgers (11, 21) ist, und
wobei die zweite Öffnung (43) durch einen Teil des anderen Sensorträgers (11, 12) oder Films bedeckt ist,
wobei die zweite Öffnung (43) vorzugsweise durch den Fensterbereich (6) des anderen Sensorträgers (11, 21) oder Films und/oder zweiten Films (1, 1') bedeckt ist, wobei die Abschnitte der Sensorträger (11, 21) oder des Films zumindest teilweise die Kammer (32) begrenzen.

16. Kassette (40) nach einem der Ansprüche 13 bis 15,
wobei das Halterelement (41) außerdem zumindest zwei Kanäle (44, 45) umfasst,
wobei der erste Kanal (44) den Einlass (33) mit der Außenoberfläche des Halterelements (41) verbindet und der zweite Kanal (45) den Auslass (34) mit der anderen Außenoberfläche des Halterelements (41) verbindet.

17. Kassette (40) nach einem der Ansprüche 13 bis 16, die zwei Halterelemente (41) umfasst, wobei
a) die Sensorträger (11, 21) und die Dichtung (31) oder
b) der Sensorträger (11) und ein Film (1') und die Dichtung (31)
zwischen den Halterelementen (41) fixiert sind,
wobei die Halterelemente (41) vorzugsweise Vorsprünge (209), insbesondere Positionierungsstifte, und Löcher (47) umfassen, die miteinander übereinstimmen, wenn die Halterelemente (41) zusammengesetzt werden, und
wobei die Sensorträger (11, 21), der Film und/oder die Dichtung (31) Positionierungslöcher (12, 22, 39) aufweisen, die von zumindest einigen der Vorsprünge (209) penetriert werden.

18. Kassette (40) nach einem der Ansprüche 13 bis 17, die zwei Halterelemente (41) umfasst, wobei die Halterelemente (41) die Anordnung (207, 227, 230, 247) komprimieren, um die Kammer (32) zu versiegeln.

19. Verfahren zur Messung einer Konzentration der Analytverbindung in einem Analytfluid (210) unter Verwendung von Nukleinsäureamplifikation mit einer Sensoranordnung (207, 227, 237, 247) oder einer Kassette (40) nach einem der vorhergehenden Ansprüche,
a) wobei ein elastischer flexibler erster Film (1) und zweiter Film (1') bereitgestellt sind, wobei eine erste Sensorverbindung (5) auf einer Region (7) des ersten Films (1) und eine zweite Sensorverbindung (5') auf einer Region (7') des zweiten Films (1') angeordnet ist, wobei die Sensorverbindungen (5, 5') auf gegenüberliegenden Sensoroberflächen (204, 204') der Filme (1,1') angeordnet sind, die einander zugewandt sind,
b) wobei die erste Sensorverbindung (5) an einen ersten Teil des Moleküls der Analytverbindung bindet oder daran haftet und die zweite Sensorverbindung (5') an einen zweiten Teil des Moleküls der Analytverbindung bindet oder daran haftet, und
c) wobei das Analytfluid (210) in einer Kammer (32) angeordnet wird, die zwischen den beiden Filmen (1, 1') ausgebildet und von diesen umgeben ist,
d) wobei der erste Film (1) und der zweite Film (1') auf eine Weise elastisch verbogen oder verformt werden, dass der Abstand zwischen den gegenüberliegenden Oberflächen der Filme (1,1'), die einander zugewandt sind, verringert wird und gleich wie oder kleiner als der Abstand zwischen dem ersten Abschnitt und dem zweiten Abschnitt des Moleküls der Analytverbindung ist,
e) sodass ein Sensor zwischen der Region (7) des ersten Films (1), auf dem eine erste Sensorverbindung (5) angeordnet ist, und der Region (7') des zweiten Films (1), auf dem die zweite Sensorverbindung (5') angeordnet ist, ausgebildet ist, wobei die Regionen (7, 7') einander zugewandt angeordnet sind, und
f) wobei die Menge an Molekülen, die an die erste und zweite Sensorverbindung (5, 5') gebunden oder angehaftet wird, gemessen wird, wobei die Kapazität oder Impedanz zwischen den elektrischen Leitern (2) in der Region (7, 7') des ersten Films (1) und jener des zweiten Films (1'), auf denen die Sensorverbindungen (5, 5') angeordnet sind, gemessen wird und die Kapazität oder Impedanz die Menge der Analytverbindung im Analytfluid (210) angibt.

20. Verfahren nach Anspruch 19, wobei Partikel (70) oder Kügelchen mit einem vorgegebenen Durchmesser zum Analytfluid (210) zugesetzt werden, wobei die Partikel (70) oder Kügelchen in die Kammer (32) zwischen den beiden Filmen (1, 1') gefüllt werden, sodass der Mindestabstand zwischen den Sensoroberflächen (204, 204') der beiden Filme (1, 1') durch den Durchmesser der Partikel definiert ist.

21. Verfahren nach einem der Ansprüche 19 bis 20 mit zwei Sensorträgern (11, 21) nach einem der Ansprüche 1 bis 8, die jeweils einen Film (1, 1') umfassen, mit einer Anordnung nach einem der Ansprüche 9 bis 13 oder einer Patrone (40) nach einem der Ansprüche 14 bis 19, welche die Filme (1, 1') enthält,
wobei eine Vielzahl von Sensoren zwischen den Regionen (7) des ersten Films (1), auf dem die erste Sensorverbindung (5) oder eine Vielzahl davon angeordnet ist, und den Regionen (7') des zweiten Films (1'), auf dem die zweite Sensorverbindung (5') oder eine Vielzahl davon angeordnet ist, ausgebildet ist, wobei die Regionen (7, 7') einander zugewandt angeordnet sind, und
wobei die Konzentration von Analytverbindungen, die sich zwischen den beiden einander zugewandten Regionen (7, 7') eines Sensors befinden oder dort abgelagert werden, separat für jeden Sensor bestimmt wird.

22. Verfahren nach einem der Ansprüche 19 bis 21, wobei vor dem Einstellen des Abstands der gegenüberliegenden Filme (1, 1') der Abstand der Filme (1, 1') erhöht wird, um die Menge an Fluid innerhalb der Kammer (32) zu erhöhen, und/oder der Abstand der Filme (1, 1') geregelt wird, um das interne Volumen der Kammer (32) einzustellen, um die Menge an Analytfluid (210) zu kontrollieren, das in der Kammer (32) aufgenommen wird.

23. Verfahren nach einem der Ansprüche 19 bis 22,
wobei der Abstand zwischen den Filmen (1, 1') vorzugsweise vor oder während der Messung wiederholt verändert wird, sodass das Analytfluid (210) in der Kammer (32) mechanisch bewegt wird, sodass durch Einstellung des Flusses in der Kammer (32) ein spezifisches Scherkraftverteilungsprofil in der Kammer (32) erreicht wird.

24. Verfahren nach einem der Ansprüche 19 bis 23, wobei der Abstand zwischen den Filmen (1, 1') verändert wird, um Fluid in die Kammer (32) oder aus der Kammer (32) zu pumpen.

## Revendications

1. Dispositif de capteur (207, 227, 237, 247) comprenant
- deux supports de capteur (11, 21), chaque support de capteur (11) comprenant un film (1, 1') ayant une pluralité d'éléments de capteur (4) fonctionnalisés, chacun comprenant une couche fonctionnelle (9) formée par un revêtement à travers lequel les composés du capteur (5) sont immobilisés, dans lequel les couches fonctionnelles (9) sont situées sur la même surface (204, 204') du film (1, 1') à l'intérieur d'une zone de fenêtre (6), dans lequel une région (7, 7') de chacune des couches fonctionnelles (9) des éléments de capteur (4) est fonctionnalisée avec un ou plusieurs composés de capteur (5, 5'), les composés de capteur (5, 5') contenant des oligonucléotides qui se lient aux sites de liaison des molécules d'analyte, chaque couche fonctionnelle ayant un revêtement individuel de fonctionnalisation et
- un joint (31) comprenant une ouverture centrale (38),
- dans lequel le film (1) du premier support de capteur (11) est flexible,
dans lequel le joint (31) est disposé entre les deux supports de capteur (11, 21), de telle sorte qu'une chambre (32) est formée entre les deux supports de capteur (11, 21) et entourée par le bord intérieur (37) du joint (31),
dans lequel les surfaces respectives des supports de capteur (11, 21), sur lesquelles les éléments de capteur (4) sont disposés, sont orientées face les unes aux autres et chaque élément de capteur (4) et/ou conducteur (2) d'un des supports de capteur (11, 21) est opposé ou fait face à au moins un ou à chaque élément de capteur et/ou conducteur (2) du support de capteur (21, 11) opposé, et
dans lequel le dispositif de capteur comprend une pluralité de conducteurs électriques (2), ayant de préférence au moins une partie en forme de ligne, chacun des conducteurs (2) étant affecté à un élément de capteur (4), et chaque conducteur (2), de préférence la partie en forme de ligne de celui-ci, étant situé en dessous de la couche fonctionnelle (9) de l'élément de capteur (4) respectif dans ou sur le film et/ou le deuxième film (1, 1').

2. Dispositif de capteur (207, 227, 237, 247) selon la revendication 1, dans lequel la matière du deuxième film (1') a un module de flexion supérieur à 5 Gpa.

3. Dispositif de capteur selon la revendication 1 ou 2, dans lequel la matière du film et/ou du deuxième film a un module de flexion de 0,5 GPa à 2 GPa, de préférence de 0,75 GPa à 1,25 GPa, et/ou
dans lequel l'épaisseur du film (1, 1') et/ou du deuxième film est entre 10 µm et 300 µm, en particulier entre 100 µm et 200 µm, et/ou
dans lequel le film (1) et/ou le deuxième film est composé d'un polymère, tel que le polyéthylène, le téréphtalate de polyéthylène (PET), le polyimide, le naphtalate de polyéthylène, le polycarbonate, le polyéthersulfone, le polyarylate et/ou des mélanges de ceux-ci.

4. Dispositif de capteur selon l'une quelconque des revendications précédentes, dans lequel
la flexibilité et l'élasticité de la matière du film (1) et/ou du deuxième film, et/ou la flexibilité et l'élasticité de la matière des couches fonctionnelles (9), et/ou la taille et/ou la forme de la zone de fenêtre (6),
sont sélectionnées de telle sorte que, lorsque la limite de la zone de fenêtre (6) est fixée dans la direction normale par rapport au plan du film et/ou du deuxième film (1, 1'), et la zone de fenêtre (6) est déviée et/ou déformée, en dehors du plan du film et/ou du deuxième film (1, 1') dans la direction normale par rapport au plan du film et/ou du deuxième film (1, 1'), dans lequel au moins une partie de la surface est poussée de 10 à 500 µm, de préférence de 20 à 200 µm, dans la direction normale par rapport au plan du film et/ou du deuxième film (1, 1'), le film et/ou le deuxième film (1, 1') est dévié élastiquement, en particulier sans être endommagé.

5. Dispositif de capteur selon l'une quelconque des revendications précédentes, dans lequel au moins la zone de fenêtre (6) du film est transparente et/ou la zone de fenêtre (6) est disposée au centre du film et/ou du deuxième film (1, 1').

6. Dispositif de capteur selon l'une quelconque des revendications précédentes, **caractérisé en ce que** une ou plusieurs entretoises (71), chacune ayant de préférence la forme d'une protubérance, sont disposées sur la surface du support de capteur (11, 21) sur le côté des couches fonctionnelles (9), les entretoises (71) étant saillantes de la surface du support de capteur (11, 21) de 0,05 µm à 1 µm, de préférence de 0,2 µm à 0,5 µm.

7. Dispositif de capteur selon l'une quelconque des revendications précédentes, **caractérisé en ce que** les composés de capteur (5) contiennent des polymères organiques ou des molécules d'ADN ou d'ARN.

8. Dispositif de capteur selon l'une quelconque des revendications précédentes,
dans lequel le support de capteur comprend de préférence un connecteur électrique (3) disposé sur le film et/ou le deuxième film (1, 1'), le connecteur électrique (3) comprenant plusieurs blocs de connecteur, chacun des blocs de connecteur étant raccordé à l'un des conducteurs (2), et/ou
dans lequel les conducteurs (2) dans ou sur le film et/ou le deuxième film (1, 1') contiennent ou sont composés de nickel, cuivre, or, carbone, oxyde d'indium-étain, semiconducteurs ou toute autre matière conductrice similaire.

9. Dispositif de capteur selon l'une quelconque des revendications précédentes,
dans lequel le film et/ou le deuxième film (1, 1') a une forme rectangulaire,
dans lequel les conducteurs (2) sont disposés en parallèle dans au moins la zone de la fenêtre (6) du film et/ou du deuxième film (1, 1'), et
dans lequel au moins une partie de chaque élément de capteur (4) et/ou chaque conducteur (2), de préférence la région fonctionnalisée (7) respective de l'élément de capteur (4), est inclinée vers les bords du film et/ou du deuxième film (1, 1'), de préférence d'un angle de 40° à 50°, en particulier 45°.

10. Dispositif de capteur selon l'une quelconque des revendications précédentes, dans lequel le deuxième film (1') comporte une pluralité de conducteurs électriques (2) en forme de ligne et un connecteur électrique (3) et/ou des entretoises (71) disposés sur sa surface.

11. Dispositif de capteur (207, 227, 237, 247) selon l'une quelconque des revendications précédentes,
dans lequel des particules (70) ou des perles sont disposées entre les surfaces opposées (204, 204') des supports de capteur à l'intérieur de la chambre (32),
dans lequel les particules (70) ou les perles sont de préférence incluses ou dispersées dans le fluide d'analyte (210) contenant les molécules d'analyte qui doivent être analysées, dans lequel la distance minimale entre les surfaces du capteur (204, 204') des deux films (1, 1') est définie par le diamètre des particules (70) ou des perles.

12. Dispositif de capteur (207, 227, 237, 247) selon l'une quelconque des revendications précédentes,
**caractérisé par** une entrée (33) et une sortie (34) amenant à travers un ou les deux supports de capteur (11, 21) et/ou dans le joint (31), amenant de l'extérieur à la chambre (32) et permettant au fluide de s'écouler dans la chambre (32) ou hors de la chambre (32) à travers l'entrée (33) et la sortie (34).

13. Cartouche (40) comprenant
- un dispositif (207, 227, 237, 247) selon l'une quelconque des revendications précédentes, et
- au moins un, de préférence deux, éléments de support (41),
dans lequel le joint (31) et les supports de capteur (11, 21) ou un support de capteur (11) et un film (1') sont fixes et compressés par les éléments de support (41) de telle sorte que la chambre (32) soit confinée par le joint (31), et/ou les supports de capteur (11, 21) et/ou le film (1, 1'), de telle sorte que la chambre (32) soit fermée et imperméable au liquide.

14. Cartouche (40) selon la revendication 13, dans laquelle l'élément de support (41) comporte au moins une ouverture (42), dans laquelle l'ouverture (42) est couverte par une partie de l'un des supports de capteur (11, 21), de préférence par la zone de fenêtre (6), ladite partie confinant au moins partiellement la chambre (32).

15. Cartouche (40) selon la revendication 13 ou 14,
dans laquelle l'élément de support (41) comporte deux ouvertures opposées (42, 43), la première ouverture (42) étant couverte par une partie de l'un des supports de capteur (11, 21), qui est de préférence la zone de fenêtre (6) dudit support de capteur (11, 21), et
la deuxième ouverture (43) étant couverte par une partie de l'autre support de capteur (11, 12) ou film,
la deuxième ouverture (43) étant de préférence couverte par la zone de fenêtre (6) de l'autre support de capteur (11, 21) ou film et/ou deuxième film (1, 1'), lesdites parties des supports de capteur (11, 21) ou du film confinant au moins partiellement la chambre (32).

16. Cartouche (40) selon l'une des revendications 13 à 15,
dans lequel l'élément de support (41) comprend également au moins deux canaux (44, 45),
dans lequel le premier canal (44) raccorde l'entrée (33) à la surface extérieure de l'élément de support (41) et le deuxième canal (45) raccorde la sortie (34) à la surface extérieure de l'élément de support (41).

17. Cartouche (40) selon l'une des revendications 13 à 16 comprenant deux éléments de support (41), dans laquelle
a) les supports de capteur (11, 21) et le joint (31), ou
b) le support de capteur (11) et un film (1') et le joint (31),
sont fixés entre les éléments de support (41),
dans lequel les éléments de support (41) comprennent de préférence des protubérances (209), en particulier des goujons, et des orifices (47), correspondant les uns aux autres quand les éléments de support (41) sont assemblés, et
dans lequel les supports de capteur (11, 21), le film et/ou le joint (31) comportent des orifices de positionnement (12, 22, 39) pénétrés par au moins certaines des protubérances (209).

18. Cartouche (40) selon l'une des revendications 13 à 17 comprenant deux éléments de support (41), les éléments de support (41) comprimant le dispositif (207, 227, 230, 247) pour fermer la chambre (32).

19. Procédé pour la mesure d'une concentration du composé d'analyte dans un fluide d'analyte (210) en utilisant l'amplification d'acide nucléique, avec un dispositif de capteur (207, 227, 237, 247) ou une cartouche (40) selon l'une des revendications précédentes,
a) dans lequel un premier film (1) élastiquement flexible et un deuxième film (1') sont disposés, un premier composé de capteur (5) étant disposé sur une région (7) du premier film (1) et un deuxième composé de capteur (5') étant disposé sur une région (7') du deuxième film (1'), les composés de capteur (5, 5') étant disposés sur les surfaces de capteur (204, 204') opposées des films (1,1') face l'une à l'autre,
b) dans lequel le premier composé de capteur (5) se lie ou adhère à une première partie de la molécule du composé d'analyte et le deuxième composé de capteur (5') se lie ou adhère à une deuxième partie de la molécule du composé d'analyte, et
c) dans lequel le fluide d'analyte (210) est disposé dans une chambre (32) formée entre et entourée par les deux films (1, 1'),
d) dans lequel le premier film (1) et le deuxième film (1') sont déviés ou déformés élastiquement de telle manière que la distance entre les surfaces opposées des films (1, 1') orientées l'une face à l'autre soit réduite et soit inférieure ou égale à la distance entre la première partie et la deuxième partie de la molécule du composé d'analyte,
e) de telle sorte qu'un capteur soit formé entre la région (7) du premier film (1) sur lequel le premier composé de capteur (5) est disposé et la région (7') du deuxième film (1) sur laquelle le deuxième composé de capteur (5') est disposé, lesdites régions (7, 7') étant disposées pour être orientées l'une face à l'autre, et
f) dans lequel la quantité de molécules liées ou collées au premier et au deuxième composés de capteur (5, 5') est mesurée, dans lequel la capacité ou l'impédance entre les conducteurs électriques (2) dans les régions (7, 7') du premier film (1) et du deuxième film (1') sur lesquelles les composés de capteur (5, 5') sont disposés est mesurée, et ladite capacité ou impédance indiquant la quantité du composé d'analyte dans le fluide d'analyte (210).

20. Procédé selon la revendication 19, dans lequel les particules (70) ou les perles d'un diamètre donné sont ajoutées au fluide d'analyte (210), dans lequel les particules (70) ou les perles sont remplies dans la chambre (32) entre les deux films (1, 1'), de telle sorte que la distance minimale entre les surfaces du capteur (204, 204') des deux films (1, 1') soit définie par le diamètre des particules.

21. Procédé selon l'une quelconque des revendications 19 et 20, avec deux supports de capteur (11, 21) selon l'une des revendications 1 à 8, chacun comprenant un film (1, 1') ou avec un dispositif selon l'une des revendications 9 à 13 ou une cartouche (40) selon l'une des revendications 14 à 19 comprenant les films (1, 1'),
dans lequel une pluralité de capteurs est formée entre les régions (7) du premier film (1) sur lequel le premier composé de capteur (5) ou la pluralité de ceux-ci est disposé et les régions (7') du deuxième film (1') sur lesquelles le deuxième composé de capteur (5') ou la pluralité de ceux-ci est disposé, lesdites régions (7, 7') étant disposées pour être orientées l'une face à l'autre, et
dans lequel la concentration des composés d'analyte situés ou déposés entre deux régions se faisant face (7, 7') d'un capteur est déterminée séparément pour chaque capteur.

22. Procédé selon l'une quelconque des revendications 19 à 21, dans lequel avant de fixer la distance des films (1, 1') opposés, la distance des films (1, 1') est accrue pour augmenter la quantité de fluide à l'intérieur de la chambre (32) et/ou
la distance des films (1, 1') est régulée pour ajuster le volume interne de la chambre (32) afin de contrôler la quantité de fluide d'analyte (210) accueillie dans la chambre (32).

23. Procédé selon l'une des revendications 19 à 22,
dans lequel la distance entre les films (1, 1') est modifiée de préférence répétitivement avant ou pendant la mesure de telle sorte que le fluide d'analyte (210) dans la chambre (32) soit agité mécaniquement, afin qu'en ajustant le débit dans la chambre (32) un profil de distribution de force de cisaillement spécifique soit obtenu dans la chambre (32).

24. Procédé selon l'une quelconque des revendications 19 à 23, dans lequel la distance entre les films (1, 1') est modifiée afin de pomper le fluide dans la chambre (32) ou hors de la chambre (32).
